# EUROPEAN PATENT APPLICATION

(11) **EP 1 514 926 A1**
(43) Date of publication of application: **16.03.2005**
(21) Application number: 03730727.9
(22) Date of filing: 30.05.2003
(51) Int. Cl.: C12N 15/09, C07K 14/47, C07K 16/18, C12N 1/15, C12N 1/19, C12N 1/21, C12N 5/10, C12P 21/02, C12Q 1/02, C12Q 1/68, G01N 33/15, G01N 33/50, G01N 33/68, C12P 21/08

(54) **INFLAMMATORY SKIN DISEASE-RELATED SLURP-2 GENE AND UTILIZATION THEREOF**

(30) Priority: 31.05.2002 JP 2002160285
(71) Applicant: Inoko, Hidetoshi, Atsugi-shi, Kanagawa 243-0034 (JP)
(72) Inventor: INOKO, Hidetoshi, Atsugi-shi, Kanagawa 243-0034 (JP); TSUJI, Hitomi, Isehara-shi, Kanagawa 259-1142 (JP); OKAMOTO, Kouichi, 1-21-3, Sakuradai, Isehara-shi, Kanagawa (JP); MATSUZAKA, Yasunari, Isehara-shi, Kanagawa 259-1133 (JP); TAMIYA, Gen, Isehara-shi, Kanagawa 259-1143 (JP)
(74) Representative: Woods, Geoffrey Corlett
(86) International application number: PCT/JP2003/006836
(87) International publication number: WO 2003/102182

(57) **Abstract**

The present inventors used microarrays to identify a novel gene from cDNA clones showing significantly different gene expression in normal tissues compared to psoriasis lesion tissues. This gene was named secretory Ly-6/uPAR-related protein-2 (SLURP-2). Quantitative real-time RT-PCR analysis using total RNA extract was used to compare SLURP-2 gene expression between psoriatic lesional skin, non-lesional skin, and normal skin, to indicate that the SLURP-2 gene is significantly upregulated in psoriasis lesions. Thus, SLURP-2 gene can be used as a diagnostic marker for inflammatory skin diseases such as psoriasis.

## Description

### Technical Field

The present invention relates to a gene associated with inflammatory skin diseases, and to uses thereof.

### Background Art

Psoriasis vulgaris (MIM 177900) is a chronic inflammatory skin disease, the prevalence rate of which is 2-3% in Caucasian populations (Nevitt, G.J., and Hutchinson, P.E. (1996) Br. J. Dermatol. 135: 533-538); 1-2% in English and European populations (Hellgren, L. (1967) Psoriasis: The Prevalence in Sex, Age and Occupational Groups in Total Populations in Sweden. Almquist & Wiksell, Stockholm.; Krueger, G.G., Bergstresser, P.R., Nicholas, J.L., and Voorhees, J.J. (1984) Psoriasis. Am. Acad. Dermatol. 11: 937-947); and 0.1-0.3% in Far Eastern (Simons, R.D. (1949) J. Invest. Dermatol. 12: 286-294) and Chinese populations (Yui Yip, S. (1984) J. Am. Acad. Dermatol. 10: 965-968). The incidence rate observed in Japanese is 0.1%, which is lower than that described above.

Most cases of psoriasis vulgaris are sporadic. Sporadic cases are characterized by inflammation triggered by skin lesions showing hyperproliferation of epidermal cells, abnormal differentiation of keratinocytes (for example, keratinocyte hyperproliferation), infiltration of activated helper T cells and monocytes, and release of proinflammatory cytokines (Menter, A., and Barker, J. N. W. N. (1991) Lancet 338: 231; Stem, R.S. (1997) Lancet, 350: 349-353).

- The cause of psoriatic lesions has been suggested to be antigens/superantigens or autoantigens provided by non-dermal inducing factors (Freedberg, IM, Eisen, AZ, Wolff, K, Goldsmith, LA, Katz, SI, Fitzpatrik, TB. (1998) Fitzpatrick's Dermatology in general medicine, 5th edition, vol. 1, 510). Moreover, many reports have clarified that T cells play a very important role in the immune pathogenesis of this disease (Gottlieb, S. L., Gilleaudeau, P., Johnson R., Estes, L., et al. (1995) The response of psoriasis to a lymphocyte-selective toxin (DAB3918IL-2) suggests a primary immune, but not keratinocyte, pathogenic basis. Nat. Med. 1: 442-447; Cooper, K. D., Voorhees, J.J., Fisher, G.J., Chan, A. K. et al. (1990) Effects of cyclosporine on immunologic mechanisms in psoriasis. J. Am. Acad. Dermatol. 23: 1318-1326; Nicolas, J. F., Cahmchick, N., Thivolet, J., Wijdenes, J., et al. (1991) CD4 antibody treatment of severe psoriasis. Lancet 338: 321; Uyemura, K., Yamamura, M., Fivenson, D. F., Modline, R.L., and Nickoloff, B.J. (1993) The cytokine network in lesional and non-lesional psoriatic skin is characterized by a T-helper type 1 cell-mediated response. J. Invest. Dermatol. 101: 701-705; Nickoloff, B.J., and Wrone-Smith, T. (1999) Injection of pre-psoriatic skin with CD4+ T cells induce psoriasis. Am. J. Pathol. 155: 145-158).

The familial occurrence of psoriasis is well recognized (Hellgren, I. (1967) Psoriasis: The Prevalence in Sex, Age, and Occupational Group in Total Population in Sweden: Morphology, Inheritance and Association with Other Skin and Rheumatic Diseases. Stockolm: Almqvist and Wiksell.). Studies of twins have shown the heritability of psoriasis to be 70-90% (Faber, E.M., and Nall, M.L. (1974) Dermatologica (Basel) 148: 1-18, 1974, Brandrup, F., Hauge, M., Henningsen, J., and Ericksen, B. (1978) Arch. Dermatol. 114: 874-878, Brandrup, F., and Green, A. (1981) Acta. Derm.-Venereol. 61: 344-346, Brandrup, F., Holm, N., Grunnet, K., Henningsen, K., and Hansen, H. (1982) Acta. Derm. Venereol. 62: 229-236, Brandrup, F. (1987) The use of twins in etologic studies of psoriasis. In Fraber, E., Nall, L., Morhenn, V. et al. (eds). Psoriasis: Proceedings of the Fourth International Symposium. Elsevier, New York, pp. 401-402, Duffy, D. L., Spelman, L.S., and Martin, N.G., (1993) J. Am. Acad. Dermatol. 29: 428-434). Such familial investigations clearly indicate that there are strong genetic factors associated with psoriatic pathogenesis (Abele, D.C., Dobson, R.L., and Graham, J.B. (1963) Arch. Dermatol. 88: 38-47, Farber, E.M., Nall, M.L., and Watson, W. (1974) Arch Dermatol. 109: 207-211, Brandrup, F., Hauge, M., Henningsen, K., and Eriksen, B. (1978) Arch Dermatol. 114: 874-878).

Several psoriasis-susceptibility loci have been reported on chromosome 6p (PSORS1 [MIM 177900]) (Trembath, R.C., et al. (1997) Hum Mol. Genet. 6: 813-820, Nair, R.P., et al. (1997) Hum. Mol. Genet. 6: 1349-1356, Oka, A. et al. (1999) Hum. Mol. Genet. 8: 2165-2170), 17q (PSORS2 [MIM 602723]) (Tomfohrde, J. et al. (1994) Science 264: 1141-1145), and 4q (PSORS3 [MIM 601454]) (Matthews, D. et al. (1996) Nat. Genet. 14: 231-233). In addition, candidate psoriasis loci have been reported on chromosomes 16q, 20p (Nair, R.P. et al. (1997) Hum. Mol. Genet. 6: 1349-1356), 8q (Brandrup, F., Hauge, M., Henningsen, K., and Eriksen, B. (1978) Arch Dermatol. 114: 874-878), 1q (PSORS4 [MIM 603935]) (Capon, F. et al, (1999) J. Invest. Dermatol. 112: 32-35), and 3q (PSORS5 [MIM 604316]) (Enlund, F. et al. (1999) Eur. J. Hum. Genet. 7: 783-790).

The Ly-6 superfamily is a group of lymphocyte antigens comprising a unique structure, comprising eight to ten conserved cysteine residues characteristically located and having a glycosyl-phosphatidyl inositol (GPI)-anchor that adheres to cell surfaces (Palfree, R. G. (1996) Ly-6 domain proteins-new insights and new members: a C-terminal Ly-6 domain in sperm acrosomal protein SP-10. Tissue antigens 48: 71-79; Low, M. G. (1989) The glycosyl-phosphatidylinositol anchor of membrane proteins. Biochim. Biophy. Acta 988: 427-454). Two subfamilies of the Ly-6 superfamily are known (Adermann, K., Wattler, F., Wattler, S., Heine, G., Meyer, M., Forssmann, W. G. and Nehls, M. (1999) Structural and phylogenetic characterization of human SLURP-1, the first secreted mammalian member of the Ly6/uPAR protein superfamily. Protein Sci. 8: 810-819). One of these is the transmembrane protein described above, and the other is a secretory protein without a GPI-anchor.

Since the time when Ly-6 superfamily members were first identified in mice (McKenzie, I. F., Gardiner, J., Cherry, M., and Snell, G. D. (1977) Lymphocyte antigens: Ly-4, Ly-6, and Ly-7. Transplant. Proc. 9: 667-669) , many family proteins have been isolated in not only mice but also humans. The human Ly-6 superfamily comprises CD59 (Bickmore, W. A., Longbottom, D., Oghene, k., Fletcher, J. M., and van Heyningen, V. (1993) Colocalization of the human CD59 gene to 11p13 with the MIC11 cell surface antigen. Genomics 17: 129-135), the urokinase-type plasminogen activator receptor (uPAR) (Suh, T. T., Nerlov, C., Dano, K., and Degen, J. L. (1994) The murine urokinase-type plasminogen activator receptor gene. J. Biol. Chem. 269: 25992-25998), prostate stem cell antigen (Reiter, R. E., Gu, Z., Watabe, T., Thomas, G., Szigeti, et al. (1998) Prostate stem cell antigen: a cell surface marker overexpressed I prostate cancer. Proc. Natl. Acad. Sci. USA 95: 1735-1740), SP-10 (Palfree, R. G. (1996) Ly-6 domain proteins-new insights and new members: a C-terminal Ly-6 domain in sperm acrosomal protein SP-10. Tissue antigens 48: 71-79), and snake venom excluding lymphocyte antigen precursor (Miwa, J. M., Ibanez-Tallon, I., Crabtree, G. W., Sanchez, R., et al. (1999) Neuron 23: 105-114). The gene loci of most members were identified on chromosome 8, in particular on 8q24.3, which is homologous to the mouse Ly-6 gene locus that was mapped to the D band of mouse chromosome 15 (Kamiura, S., Nolan, C.M., and Meruelo, D. (1992) Long-range physical map of the Ly-6 complex: mapping the ly-6 multigene family by field-inversion and two-dimensional gel electrophoresis. Genomics 12: 89-105). The functions of most human Ly-6 superfamily members have yet to be clarified.

### Disclosure of the Invention

The present invention was made in view of the above circumstances. An objective of the present invention is to isolate and identify novel genes associated with inflammatory skin diseases, and to provide methods for using the same. More specifically, an objective of the present invention is to provide novel psoriasis-associated genes, and methods for using the same.

In order to identify novel psoriasis-associated genes, the present inventors used microarrays to comprehensively analyze transcriptional changes occurring in lesional and non-lesional areas of skin from patients with psoriasis, and compared these with healthy subjects. As a result, the present inventors found 86 ESTs that were upregulated or downregulate by about ten-fold in affected psoriatic lesions, compared with normal tissues. The present inventors isolated a full-length cDNA corresponding to one of the ESTs, using RACE and oligo-capping methods. A homology search of the cDNA revealed that the protein encoded by the cDNA is a new member of the Ly-6 /uPAR superfamily. The present inventors named the protein encoded by the cDNA the "secretory Ly-6/uPAR-related protein-2" (SLURP-2). Expression analysis using RT-PCR verified that the SLURP-2 gene is expressed in various tissues, mainly in epithelial tissues. In addition, while the SLURP-2 gene was not detected in bone marrow, nor in spleen, it was detected in the thymus. Quantitative real-time RT-PCR using total RNA extracts was carried out to compare gene expression between psoriatic, non-psoriatic, and normal skin. The results showed that the SLURP-2 gene is significantly upregulated in affected psoriatic skin. Thus, the SLURP-2 gene can be used as a diagnostic marker for inflammatory skin diseases, such as psoriasis.

Accordingly, this invention relates to genes associated with inflammatory skin diseases, and use of the same. More specifically, this invention provides:
[1] a DNA of any one of (a) to (e):
   (a) a DNA encoding a polypeptide comprising the amino acid sequence of SEQ ID NO: 2;
   (b) a DNA comprising the coding region of the nucleotide sequence of SEQ ID NO: 1;
   (c) a DNA encoding a polypeptide comprising the amino acid sequence of SEQ ID NO: 2, wherein one or more amino acids are substituted, deleted, inserted, and/or added, and wherein said polypeptide is functionally equivalent to the polypeptide comprising the amino acid sequence of SEQ ID NO: 2;
   (d) a DNA that hybridizes under stringent conditions to a DNA comprising the nucleotide sequence of SEQ ID NO: 1, and that encodes a polypeptide functionally equivalent to a polypeptide comprising the amino acid sequence of SEQ ID NO: 2; and
   (e) a DNA that hybridizes under stringent conditions to a DNA comprising the nucleotide sequence of SEQ ID NO: 1;
[2] a DNA encoding a partial peptide of a polypeptide comprising the amino acid sequence of SEQ ID.NO: 2;
[3] a polypeptide encoded by the DNA of [1] or [2];
[4] a vector into which the DNA of [1] or [2] is inserted;
[5] a transformed cell comprising the DNA of [1] or [2], or the vector of [4];
[6] a method for producing the polypeptide of [3], wherein said method comprises the steps of:
   culturing the transformed cell of [5]; and
   recovering the expressed polypeptide from said transformed cell, or culture supernatant thereof;
[7] an antibody binding to the polypeptide of [3];
[8] the antibody of [7], which is a monoclonal antibody;
[9] an oligonucleotide comprising at least 15 nucleotides, which is complementary to the DNA of [1] or a complementary strand thereof;
[10] a method of screening for a compound that binds to the polypeptide of [3], wherein said method comprises the steps of:
   (a) contacting a test sample with said polypeptide;
   (b) detecting a binding activity between said polypeptide and said test sample; and
   (c) selecting a compound having binding activity to said polypeptide;
[11] a method of testing for an inflammatory skin disease, wherein said method comprises the steps of:
   (a) preparing an RNA sample from a test subject;
   (b) measuring, in said RNA sample, the amount of RNA that encodes the polypeptide of [3]; and
   (c) comparing the amount of RNA thus determined with a control sample;
[12] a method of testing for an inflammatory skin disease, which comprises the steps of:
   (a) preparing a cDNA sample from a test subject;
   (b) measuring, in said cDNA sample, the amount of a cDNA that encodes the polypeptide of [3]; and
   (c) comparing the amount of cDNA thus determined with a control sample;
[13] a method of testing for an inflammatory skin disease, wherein said method comprises the steps of:
   (a) preparing a polypeptide sample from a test subject;
   (b) measuring the amount of the polypeptide of [3] in said polypeptide sample; and
   (c) comparing the amount of polypeptide thus determined with a control sample;
[14] the method of any one of [11] to [13], wherein the inflammatory skin disease is psoriasis;
[15] a test agent for an inflammatory skin disease, comprising the oligonucleotide of [9];
[16] a test agent for an inflammatory skin disease, comprising the antibody of [7] or [8]; and
[17] the test agent of [15] or [16], wherein the inflammatory skin disease is psoriasis.

This invention provides a novel "secretory Ly-6/uPAR-related protein-2 (SLURP-2) gene". The cDNA sequence of the human SLURP-2 gene and the amino acid sequence of the polypeptide encoded by the cDNA are shown in SEQ ID NOs: 1 and 2, respectively.

The SLURP-2 gene was isolated as a gene expressed in inflammatory skin disease-affected areas. Therefore, this gene can be used as a diagnostic marker for inflammatory skin diseases. In addition, this gene is expected to be applicable to the prevention and therapy of inflammatory skin diseases.

According to the present invention, the term "inflammatory skin disease" refers to diseases characterized by occurrence of a skin lesion resulting from infiltration of inflammatory cells such as activated helper T cells and monocytes, due to acanthosis, or due to abnormal differentiation of keratinocytes. According to the present invention, inflammatory skin diseases comprise, for example, psoriasis, lichen planus, pityriasis rubra pilaris, and palmoplanter pustulosis, but are not limited thereto. While the term "psoriasis" generally means psoriasis vulgaris, psoriasis in the present invention includes psoriasis pustulosa, psoriasis arthropatica, and other psoriases, in addition to psoriasis vulgaris.

The present invention also comprises DNAs encoding polypeptides functionally equivalent to those comprising the amino acid sequence of SEQ ID NO: 2. Such DNAs comprise, for example, DNAs that encode mutants, allelic variants, and homologs of a polypeptide comprising the amino acid sequence of SEQ-ID NO: 2. A "functionally equivalent" polypeptide as used herein denotes that the polypeptide comprises biological and biochemical functions equivalent to those of SLURP-2. In addition, SLURP-2 in the present invention preferably comprises the property of increased expression in a lesional area as compared to a non-lesional area. Methods known to those skilled in the art can be used to determine whether a polypeptide encoded by a target DNA comprises such a property.

A DNA encoding a polypeptide functionally equivalent to a target polypeptide may be prepared by, as another method well known to those skilled in the art, a hybridization technique (Sambrook, J et al., Molecular Cloning 2nd ed., 9.47-9.58, Cold Spring Harbor Lab. press, 1989). In other words, techniques used to isolate DNAs highly homologous to a DNA sequence encoding SLURP-2 (SEQ ID NO: 1) or its portion, and techniques that use this DNA to isolate polypeptides functionally equivalent to SLURP-2, are well known to those skilled in the art.

This invention comprises DNAs that hybridize under stringent conditions to a DNA encoding SLURP-2, and DNAs that encode polypeptides functionally equivalent to SLURP-2. Such DNAs comprise, for example, homologous DNAs derived from humans, monkeys, mice, rats, pigs, and cows, but are not limited thereto.

Those skilled in the art can select suitable hybridization conditions for isolating DNAs encoding polypeptides functionally equivalent to SLURP-2. Such hybridization conditions include, for example, conditions of low stringency. Examples of conditions of low stringency include post-hybridization washing in 5x SSC and 0.1% SDS at 42°C, and preferably in 5x SSC and 0.1% SDS at 50°C. More preferable hybridization conditions include those of high stringency. Highly stringent conditions include, for example, washing in 0.1x SSC and 0.1% SDS at 65°C. In these conditions, the higher the temperature, the higher the expectation of efficiently obtaining DNAs with high homology. However, several factors, including temperature and salt concentration, can influence hybridization stringency, and one skilled in the art can suitably select these factors to accomplish similar stringencies.

In addition, gene amplification methods such as the polymerase chain reaction (PCR) method, which use primers that are synthesized based on the sequence information of a DNA encoding SLURP-2 (SEQ ID NO: 1), can also be utilized to isolate DNAs encoding polypeptides functionally equivalent to SLURP-2.

A polypeptide functionally equivalent to SLURP-2, and encoded by a DNA isolated using an above hybridization or gene amplification technique, normally comprises high homology to the amino acid sequence of SLURP-2. The polypeptides of the present invention also comprise polypeptides that are functionally equivalent to SLURP-2 and are highly homologous to the amino acid sequence of the polypeptides. "Highly homologous" normally refers to sequence identity, at the amino acid level, of at least 50% or higher, preferably 75% or higher, more preferably 85% or higher, and most preferably 95% or higher.

The degree of homology of one amino acid sequence or nucleotide sequence to another can be determined by following the algorithm BLAST by Karlin and Altschl (Proc. Natl. Acad. Sci. USA, 90: 5873-5877, 1993). Programs such as BLASTN and BLASTX were developed based on this algorithm (Altschul et al. J. Mol. Biol.215: 403-410, 1990). To analyze a nucleotide sequence according to BLASTN, based on BLAST, the parameters are set as score= 100 and word length= 12, for example. On the other hand, parameters used for the analysis of amino acid sequences by BLASTX based on BLAST include, for example, score= 50 and word length= 3. The default parameters of each program are used when using BLAST and Gapped BLAST programs. Specific techniques for such analysis are known in the art (http://www.ncbi.nlm.nih.gov.)

In addition, this invention also comprises polypeptides functionally equivalent to SLURP-2 polypeptides and comprising the amino acid sequence of SLURP-2, in which one or more amino acids is mutated. Such amino acid mutations may occur naturally. The number of amino acids that may be mutated is generally 30 or less, preferably 15 or less, more preferably five or less (for example, three or less), and most preferably one or two amino acids.

An amino acid residue is preferably mutated into an amino acid residue that conserves the properties of the amino acid side chain. Examples of amino acid side chain properties are: hydrophobic amino acids (A, I, L, M, F, P, W, Y, V), hydrophilic amino acids (R, D, N, C, E, Q, G, H, K, S, T), and amino acids comprising the following side chains: aliphatic side chains (G, A, V, L, I, P); hydroxyl group-containing side chains (S, T, Y); sulfur atom-containing side chains (C, M); carboxylic acid- and amide-containing side chains (D, N, E, Q); base-containing side chains (R, K, H); and aromatic-containing side chains (H, F, Y, W) (the letters within parenthesis are the one-letter amino acid codes).

It is well known that a polypeptide comprising a modified amino acid sequence in which one or more amino acid residues is deleted, added, and/or substituted can retain its original biological activity (Mark D.F. et al., Proc. Natl. Acad. Sci. U.S.A. 81: 5662-5666 (1984); Zoller M.J. and Smith M., Nucleic Acids Res. 10: 6487-6500 (1982); Wang A. et al. , Science 224: 1431-1433; Dalbadie-McFarland G. et al., Proc. Natl. Acad. Sci. U.S.A. 79: 6409-6413 (1982)).

In addition, one method well known to those skilled in the art for preparing polypeptides functionally equivalent to a specific polypeptide is to introduce mutations into the polypeptide. For example, one skilled in the art can prepare polypeptides functionally equivalent to a SLURP-2 polypeptide by introducing appropriate mutations into the SLURP-2 amino acid sequence through site-specific mutagenesis or the like (Hashimoto-Gotoh, T. et al. (1995) Gene 152, 271-275; Zoller, MJ, and Smith, M. (1983) Methods Enzymol. 100, 468-500; Kramer, W. et al. (1984) Nucleic Acids Res. 12, 9441-9456; Kramer W, and Fritz HJ (1987) Methods. Enzymol. 154, 350-367; Kunkel, TA (1985) Proc Natl Acad Sci U S A. 82, 488-492; Kunkel (1988) Methods Enzymol. 85, 2763-2766).

Polypeptides in which amino acid residues have been added to the SLURP-2 amino acid sequence comprise fusion proteins comprising SLURP-2 polypeptides. The present invention comprises fusion proteins in which SLURP-2 polypeptides and other polypeptides are fused. Fusion proteins can be produced by, for example, ligating a DNA encoding SLURP-2 and a DNA encoding another polypeptide in frame; introducing the resulting construct into an expression vector; and then expressing this in a host cell. The polypeptides to be fused to a polypeptide of the present invention are not particularly limited.

Known peptides are, for example, FLAG (Hopp, T.P. et al., Biotechnology (1988) 6, 1204-1210), 6xHis containing six His (histidine) residues, 10xHis, Influenza agglutinin (HA), human c-myc fragment, VSP-GP fragment, p18HIV fragment, T7-tag, HSV-tag, E-tag, SV40T antigen fragment, lck tag, α-tubulin fragment, B-tag, and Protein C fragment, and can be used as peptides that are fused to a polypeptide of the present invention. Examples of polypeptides that are fused to a polypeptide of the present invention are GST (glutathione-S-transferase), Influenza agglutinin (HA), immunoglobulin constant region, β-galactosidase, and MBP (maltose-binding protein). Fusion polypeptides can be prepared by fusing a commercially available DNA encoding these polypeptides with a DNA encoding a polypeptide of the present invention; and then expressing the fused DNA thus prepared.

Any type of DNA can be used, such as a cDNA synthesized from mRNA, a genomic DNA, or a synthetic DNA, so long as the DNA encodes a polypeptide of the present invention. Also, so long as the DNAs can encode a polypeptide of the present invention, DNAs comprising arbitrary sequences based on genetic code degeneracy are also included.

The DNAs of the present invention can be prepared by methods known in the art. For example, naturally-occuring DNAs can be prepared by constructing a cDNA library from cells expressing a polypeptide of the present invention; and then carrying out hybridization using a portion of a DNA sequence of the present invention as a probe (for example, SEQ ID NO: 1) . cDNA libraries may be prepared, for example, according to methods described in the literature (Sambrook J. et al. Molecular Cloning, Cold Spring Harbor Laboratory Press (1989)). Alternatively, commercially available cDNA libraries may be used. The DNAs of the present invention can be obtained by preparing RNAs from cells that express a polypeptide of the present invention, synthesizing cDNAs using a reverse transcriptase, synthesizing oligo-DNAs based on a DNA sequence of the present invention (for example, SEQ ID NO: 1), and amplifying a cDNA encoding a polypeptide of the present invention by PCR using the oligo-DNA as a primer.

The nucleotide sequence of an obtained cDNA is determined in order to find its open reading frame, and the amino acid sequences of the polypeptides of the invention can thus be obtained. An obtained cDNA may also be used as a probe for screening a genomic library to isolate genomic DNA.

More specifically, mRNA may first be isolated from cells, tissues (such as the brain, lung, esophagus, stomach, small intestine, colon, rectum, testis, uterus, uterine cervix, thymus, skin, fetal skin, and epidermal keratinocyte), or a diseased part of an inflammatory dermatosis in which a polypeptide of the present invention is expressed. Known methods can be used to isolate mRNAs. For instance, total RNA can be prepared by guanidine ultracentrifugation (Chirgwin J.M. et al. Biochemistry 18:5294-5299 (1979)) or AGPC methods (Chomczynski P. and Sacchi N. Anal. Biochem. 162:156-159 (1987)), and mRNA can be purified from total RNA using an mRNA Purification Kit (Pharmacia) or such. Alternatively, mRNA may be directly prepared using a QuickPrep mRNA Purification Kit (Pharmacia).

The obtained mRNAs are used to synthesize cDNAs using reverse transcriptase. cDNAs may be synthesized using a kit such as the AMV Reverse Transcriptase First-strand cDNA Synthesis Kit (Seikagaku Kogyo). Alternatively, cDNAs may be synthesized and amplified following the 5'-RACE method (Frohman M.A. et al., Proc. Natl. Acad. Sci. U.S.A. 85: 8998-9002 (1988); Belyavsky A. et al., Nucleic Acids Res. 17: 2919-2932 (1989)), using the 5'-Ampli FINDER RACE Kit (Clontech) and polymerase chain reaction (PCR).

Target DNA fragments are prepared from the PCR products and linked to vector DNAs. The recombinant vectors are used to transform *E. coli* and such, and the desired recombinant vectors are prepared from selected colonies. The nucleotide sequences of the target DNAs can be verified by conventional methods, such as dideoxynucleotide chain termination.

By considering the frequency of codon usage in a host used for expression, the sequence of the DNA of the present invention can be designed for more efficient expression (Grantham R. et al., Nucleic Acids Res. 9: 43-74 (1981)). The DNAs of the present invention may be altered using a commercially available kit or by conventional methods. For instance, the DNAs may be altered by digestion with restriction enzymes, insertion of a synthetic oligonucleotide or an appropriate DNA fragment, addition of a linker, or insertion of an initiation codon (ATG) and/or stop codon (TAA, TGA, or TAG), etc.

In addition, the present invention comprises DNAs that hybridize under stringent conditions to the DNA encoding SLURP-2 (SEQ ID NO: 1). The DNAs that hybridize to the SLURP-2 gene under stringent conditions can be used to measure expression levels of the SLURP-2 gene. Therefore, DNAs that hybridize to the SLURP-2 gene under stringent conditions do not always need to encode a polypeptide. Preferably, such DNAs hybridize to a coding region of the nucleotide sequence of SEQ ID NO: 1. In addition, the DNAs preferably comprise 100 or more nucleotides, more preferably 200 or more nucleotides, and most preferably 250 or more nucleotides.

The stringency conditions described above can be also used for hybridization. DNAs hybridizing under such stringent conditions typically have a high sequence identity (for example, an identity of 70% or higher, preferably 80% or higher, more preferably 90% or higher).

The present invention provides polypeptides encoded by the DNAs of the present invention. Depending on the cell or host used to produce it or the purification method utilized (described below), the polypeptides of the present invention may have variations in their amino acid sequence, molecular weight, isoelectric point, the presence or absence of sugar chains, form, etc. Nevertheless, as long as the obtained polypeptide comprises a function equivalent to SLURP-2, it is included in the scope of the present invention. For example, if a polypeptide of the present invention is expressed in a prokaryotic cell, such as *E. coli,* the polypeptide comprises a methionine residue at the N-terminus in addition to the natural amino acid sequence of the same. Such polypeptides are also comprised as polypeptides of the present invention.

The polypeptides of the present invention can be prepared as recombinant polypeptides or as naturally occurring polypeptides, using methods commonly known in the art. Recombinant proteins can be produced by inserting DNAs encoding the polypeptides of the present invention into an appropriate expression vector, collecting the transformant obtained after introducing the vector into an appropriate host cell, obtaining an extract, and then purifying using ion exchange, reverse phase, gel filtration, or affinity chromatography. Affinity chromatography may be carried out using a column in which an antibody against a polypeptide of the present invention is fixed. A combination of such columns may also be used.

Alternatively, when the polypeptides of the present invention are expressed in host cells (e.g., animal cells or *E. coli*) as fusion polypeptides with glutathione S transferase proteins, or recombinant polypeptides with multiple histidine residues, the expressed recombinant polypeptides can be purified using a glutathione column or nickel column. After the fusion polypeptides are purified, if necessary, regions of the fusion polypeptides (other than the desired polypeptides) can be digested and removed with thrombin, factor Xa, and so on.

Naturally-occurring polypeptides of the present invention can be isolated by methods well known in the art. For example, affinity columns to which antibodies binding to the polypeptides of the present invention are bound can be employed to purify an extract of tissues or cells that express a polypeptide of the present invention (as described below). The antibodies may be polyclonal or monoclonal antibodies.

The present invention also comprises partial peptides of the polypeptides of the present invention. The partial peptides of the present invention can be used, for example, for generating antibodies against the polypeptides of the present invention, screening for compounds binding to the polypeptides of the present invention, or screening for stimulators or inhibitors of the polypeptides of the present invention. Additionally, they may be antagonists or competitive inhibitors of the polypeptides of the present invention.

When used as immunogens, the partial peptides of the present invention comprise at least seven or more, preferably eight or more, and more preferably nine or more amino acids. When used as competitive inhibitors of the polypeptides of the present invention, they may comprise at least 100 or more, preferably 200 or more, and more preferably 300 or more amino acids (for example, 400 or more amino acids) .

The partial peptides of the present invention can be produced by genetic engineering methods, known peptide synthesis methods, or by cutting the polypeptides of the present invention with appropriate peptidases. Synthesis of the peptides may be conducted according to, for example, solid or liquid phase synthesis methods.

The present invention also provides a vector into which a DNA of the present invention is inserted. The vectors of the present invention are useful in maintaining the DNAs of the present invention within host cells, or expressing the polypeptides of the present invention.

When *E. coli* is used as a host cell, there is no limitation other than that the vector should have an "ori" to amplify and mass-produce the vector in *E. coli* (e.g., JM109, DH5α, HB101, or XL1Blue, and such) , and a marker gene for selecting the transformed *E. coli* (e.g., a drug-resistance gene selected by a drug, such as ampicillin, tetracycline, kanamycin, or chloramphenicol).

For example, M13-series vectors, pUC-series vectors, pBR322, pBluescript, pCR-Script, and such can be used. Besides the vectors, pGEM-T, pDIRECT, pT7, and such can be also used for subcloning and excision of the cDNA.

When a vector is used to produce the polypeptides of the present invention, an expression vector is especially useful. When the expression vector is expressed, for example, in *E. coli*, it should comprise the above characteristics in order to be amplified in *E. coli.* Additionally, when *E. coli,* such as JM109, DH5α, HB101, or XL1-Blue, are used as host cells, the vector should have a promoter that allows efficient expression of the desired gene in *E. coli,* e.g. lacZ promoter (Ward et al. (1989) Nature 341:544-546; (1992) FASEB J. 6:2422-2427), araB promoter (Better et al. (1988) Science 240:1041-1043), or T7 promoter. Other examples of the vectors comprise pGEX-5X-1 (Pharmacia), "QIAexpress system" (QIAGEN), pEGFP, and pET (for this vector, BL21, a strain expressing T7 RNA polymerase, is preferably used as the host).

Furthermore, the vector may comprise a signal sequence for polypeptide secretion. When producing polypeptides into the periplasm of *E. coli*, the pelB signal sequence (Lei S.P. et al. J. Bacteriol. 169:4379 (1987)) may be used as a signal sequence for protein secretion. Calcium chloride methods or electroporation may be used to introduce the vector into host cells.

In addition to *E. coli,* expression vectors derived from mammals (e.g., pCDNA3 (Invitrogen), pEGF-BOS (Nucleic Acids Res. (1990) 18(17): 5322), pEF, pCDM8); insect cells (e.g., "Bac-to-BAC baculovirus expression system" (GIBCO-BRL), pBacPAK8); plants (e.g. pMH1, pMH2); animal viruses (e.g., pHSV, pMV, pAdexLcw); retroviruses (e.g. pZIPneo) ; yeasts (e.g., "Pichia Expression Kit" (Invitrogen), pNV11, SP-Q01); and *Bacillus subtilis* (e.g. pPL608, pKTH50) may also be employed to produce the polypeptides of the present invention.

In order to express proteins in animal cells, such as CHO, COS, and NIH3T3 cells, the vector must comprise a promoter necessary for expression in such cells (e.g., SV40 promoter (Mulligan et al. (1979) Nature 277: 108), MMLV-LTR promoter, EF1α promoter (Mizushima et al. (1990) Nucleic Acids Res. 18: 5322) and CMV promoter). It is preferable that the vector also comprises a marker gene for selecting transformants (for example, a drug-resistance gene selected by a drug such as neomycin and G418). Examples of vectors with such characteristics include pMAM,pDR2, pBK-RSV, pBK-CMV, pOPRSV, pOP13, and such.

Furthermore, when aiming to stably express the gene and amplify its copy number in cells, methods can be used that, for example, introduce CHO cells defective in nucleic acid synthetic pathways with a vector (such as pCHOI) carrying a DHFR gene that compensates for the defect, and then amplify the vector with methotrexate (MTX). Alternatively, when aiming for transient gene expression, examples of methods include those, in which COS cells that comprise a gene that expresses the SV40 T antigen in the chromosome are transformed with a vector (such as pcD) carrying an SV40 replication origin. The replication origin may be that of a polyomavirus, adenovirus, bovine papilloma virus (BPV), or the like. Also, to amplify the gene copy number in the host cells; selection markers, such as the aminoglycoside transferase (APH) gene, thymidine kinase (TK) gene, *E. coli* xanthine-guanine phosphoribosyl transferase (Ecogpt) gene, and the dihydrofolate reductase (dhfr) gene, may be comprised in the expression vector.

DNAs of the present invention can be expressed in animals by, for example, inserting a DNA of the invention into an appropriate vector and introducing this vector into a living cell via retroviral methods, liposome methods, cationic liposome methods, adenovirus methods, and such. Thus, it is possible to perform gene therapy for diseases caused by a mutation of the SLURP-2 gene of the present invention. The vectors used in these methods comprise, but are not limited to, adenovirus vectors (e.g., pAdexlcw), retrovirus vectors (e. g. pZIPneo), and such. General gene manipulation techniques, such as inserting a DNA of the present invention into a vector, can be performed according to conventional methods (Molecular Cloning, 5.61-5.63). Administration to a living body may be performed by ex vivo or in vivo methods.

The present invention also provides host cells into which a vector of the present invention has been introduced. The host cells into which a vector of the present invention is introduced are not particularly limited. For example, *E. coli* and various animal cells can be used. A host cell of the present invention can be used, for example, as a production system to produce and express a polypeptide of the present invention. The systems for producing the polypeptides comprise *in vitro* and *in vivo* systems. Production systems that use eukaryotic cells or prokaryotic cells are examples of *in vitro* production systems.

Eukaryotic host cells that can be used are, for example, animal cells, plant cells, and fungi cells. Mammalian cells, for example, CHO (J. Exp. Med. (1995) 108:945), COS, 3T3, myeloma, BHK (baby hamster kidney), HeLa, Vero, amphibian cells (e.g., Xenopus oocytes (Valle et al. (1981) Nature 291:358-340), and insect cells (e.g. Sf9, Sf21, Tn5) are known as animal cells. Among CHO cells, those defective in the DHFR gene, dhfr-CHO (Proc. Natl. Acad. Sci. USA (1980) 77:4216-4220) and CHO K-1 (Proc. Natl. Acad. Sci. USA (1968) 60:1275) are particularly preferable.- Among animal cells, CHO cells are particularly preferable for large-scale expression. A vector can be introduced into a host cell by, for example, calcium phosphate methods DEAE-dextran methods, methods using cationic liposome DOTAP (Boehringer-Mannheim), electroporation methods, and lipofection methods.

Plant cells originating from *Nicotiana tabacum* are known as polypeptide-producing systems and may be used as callus cultures. As fungal cells, yeast cells such as *Saccharomyces,* including *Saccharomyces cerevisiae,* or filamentous fungi such as *Aspergillus,* including *Aspergillus niger,* are known and included in the scope of this invention.

Useful prokaryotic cells comprise bacterial cells. Examples of bacterial cells include *E. coli,* for example, JM109, DH5α, HB101 and such, and *Bacillus subtilis* are also known.

These cells are transformed by a desired DNA, and the transformants are cultured in vitro to obtain a polypeptide. Transformants can be cultured using known methods. For example, the culture medium for animal cells may be a culture medium such as DMEM, MEM, RPMI1640, or IMDM, and may be used with or without serum supplements such as fetal calf serum (FCS). The pH of the culture medium is preferably between about 6 and 8. Such cells are typically cultured at about 30 to 40°C for about 15 to 200 hours, and the culture medium may be replaced, aerated, or stirred if necessary.

Production systems using animal and plant hosts may be used as systems for producing polypeptides *in vivo*. For example, target DNAs are introduced into these animal or plant hosts, polypeptides are produced in the body of the animal or plant, and then recovered. These animals and plants are included in the "hosts" of the present invention.

The animals to be used for the production system described above comprise mammals and insects. Mammals such as goats, pigs, sheep, mice, and cattle may be used (Vicki Glaser, SPECTRUM Biotechnology Applications (1993)). Alternatively, the mammals may be transgenic animals.

For instance, a target DNA may be prepared as a fusion gene with a gene that encodes a polypeptide specifically produced in milk, such as the goat β casein gene. DNA fragments comprising the fusion gene are injected into goat embryos, which are then introduced back to female goats. Target polypeptides are recovered from the milk produced by the transgenic goats (i.e., those goats born from the goats that received the modified embryos) or from their offspring. Appropriate hormones may be administered to increase the volume of milk comprising the polypeptides produced by transgenic goats (Ebert K.M. et al. (1994) Bio/Technology 12: 699-702).

Alternatively, insects, such as silkworms, may be used as hosts. Baculoviruses, into which a DNA encoding a target polypeptide has been inserted, can be used to infect silkworms, and the desired polypeptide can be recovered from body fluids (Susumu M. et al. (1985) Nature 315: 592-594).

In addition, when using plants, tobacco, for example, can be used. When using tobacco, a DNA encoding a desired polypeptide may be inserted into a plant expression vector, such as pMON 530, which is introduced into bacteria, such as *Agrobacterium tumefaciens.* Then, the bacteria are used to infect tobacco, such as *Nicotiana tabacum,* and the desired polypeptide is recovered from the leaves (Julian K. -C. Ma et al. (1994) Eur. J. Immunol. 24: 131-138).

A polypeptide of the present invention, obtained as above, may be isolated from the inside or outside (the medium and such) of host cells, and purified as a substantially pure homogeneous polypeptide. Methods for isolating and purifying polypeptides are not limited to any specific method; in fact, any standard method for isolating and purifying polypeptides may be used. For instance, column chromatography, filters, ultrafiltration, salting out, solvent precipitation, solvent extraction, distillation, immunoprecipitation, SDS-polyacrylamide gel electrophoresis, isoelectric point electrophoresis, dialysis, and recrystallization may be appropriately selected and combined to isolate and purify the polypeptides.

Chromatography such as affinity chromatography, ion-exchange chromatography, hydrophobic chromatography, gel filtration chromatography, reverse phase chromatography and adsorption chromatography may be used (Strategies for Protein Purification and Characterization: A Laboratory Course Manual. Ed. Daniel R. Marshak et al., Cold Spring Harbor Laboratory Press (1996)). These chromatographies may be performed using liquid chromatographies, such as HPLC and FPLC. Thus, the present invention provides highly purified polypeptides produced by the above methods.

A polypeptide may be optionally modified or partially deleted by treatment with an appropriate protein-modifying enzyme before or after purification. For example, trypsin, chymotrypsin, lysylendopeptidase, protein kinase, glucosidase, and such can be used as protein-modifying enzymes.

Examples of antibodies that bind to the polypeptides of the present invention are monoclonal antibodies (comprising full-length monoclonal antibodies), polyclonal antibodies, or mutants thereof.

Herein, a "monoclonal antibody" of the present invention refers to an antibody obtained from a group of substantially homogeneous antibodies, i.e., an antibody group wherein the antibodies constituting the group are homogeneous except for naturally occurring mutants that exist in a small amount. A monoclonal antibody is highly specific and interacts with a single antigenic site. Furthermore, each monoclonal antibody targets a single antigenic determinant (epitope) on an antigen, as compared to commonly used antibody preparations that typically contain various antibodies against diverse antigenic determinants (polyclonal antibodies) . In addition to their specificity, monoclonal antibodies are advantageous in that they are produced from hybridoma cultures not contaminated with other immunoglobulins. The qualifier "monoclonal" indicates the characteristics of antibodies obtained from a substantially homogeneous group of antibodies, and does not specify antibodies to be produced by a particular method. For example, the monoclonal antibodies used in the present invention can be produced by, for example, hybridoma methods, as described below (Kohler G. and Milstein C., Nature 256:495-497, 1975), or recombination methods (U.S. patent No. 4,816,567). The monoclonal antibodies used in the present invention can also be isolated from a phage antibody library (Clackson T. *et al.,* Nature 352:624-628, 1991; Marks J. D. *et al.,* J. Mol. Biol. 222:581-597, 1991). The monoclonal antibodies of the present invention particularly comprise "chimeric" antibodies (immunoglobulins) wherein a part of the heavy chain and/or light chain is derived from a specific species, or a specific antibody class or subclass, and the remaining portion of the chain from another species, or another antibody class or subclass. Furthermore, antibody fragments thereof are also comprised in the present invention; so long as they comprise a desired biological activity (U.S. Patent No. 4,816,567; Morrison S.L. *et al.,* Proc. Natl. Acad. Sci. USA 81:6851-6855, 1984).

In the present invention, "mutant antibody" refers to antibodies with amino acid sequence variations, in which one or more amino acid residues are altered. A "mutant antibody" of the present invention comprises variously altered amino acid variants, as long as they have the same binding specificity as the original antibody. Such mutants have less than 100% homology or similarity to an amino acid sequence that has at least 75%, more preferably at least 80%, even more preferably at least 85%, still more preferably at least 90%, and most preferably at least 95% amino acid sequence homology or similarity to the amino acid sequence of the variable domain of a heavy chain or light chain of an antibody.

Polyclonal antibodies are preferably produced in non-human mammals by multiple subcutaneous (sc) or intraperitoneal (ip) injections of related antigens and adjuvants. A related antigen may be bound to a protein that is immunogenic to the immunized species, for example, keyhole limpet hemocyanin, serum albumin, bovine thyroglobulin or soybean trypsin inhibitor, using bifunctional agents or inducers, for example, maleimidebenzoylsulfosuccinimide ester (binding via a cysteine residue), N-hydroxysuccinimide (via a lysine residue), glutaraldehyde, succinic anhydride, thionylchloride or R¹N=C=CR (wherein, R and R¹ are different alkyl groups).

For example, an animal is immunized with an antigen, an immunogenic conjugate or a derivative, by multiple intradermal injections of a solution containing 100 µg or 5 µg of a protein or conjugate (for a rabbit or mouse respectively), with three volumes of Freund's complete adjuvant. One month later, a booster is applied to the animal at several sites, by subcutaneous injections of 1/5 to 1/10 volume of the original peptide or conjugate in Freund's complete adjuvant. Blood is collected from the animal after seven to 14 days, and serum is analyzed for antibody titer. Preferably, a conjugate of the same antigen, but bound to a different protein and/or bound via a different cross-linking reagent, is used as the booster. A conjugate can also be produced by protein fusion through recombinant cell culture. Moreover, in order to enhance the immune response, agglutinins, such as alum, are preferably used. A selected mammalian antibody usually comprises a considerably strong binding affinity for the antigen. Antibody affinity can be determined by saturation bonding, enzyme-linked immunosorbent assays (ELISA) and competitive analysis (for example, radioimmunoassays).

As a method of screening for desired polyclonal antibodies, conventional cross-linking analysis described in "Antibodies, A Laboratory Manual" (Harlow and David Lane eds., Cold Spring Harbor Laboratory, 1988) can be performed. Alternatively, for example, epitope mapping (Champe *et al*., J. Biol. Chem. 270:1388-1394, 1995) may be performed. Preferred methods for measuring the efficacy of a polypeptide or antibody are those methods that use quantification of antibody binding affinity. In addition, other embodiments include methods wherein one or more of an antibody's biological properties are evaluated instead of antibody binding affinity. These analytical methods are particularly useful in that they indicate the therapeutic efficacy of an antibody. Often, but not always, antibodies shown to have improved properties by such analysis have also enhanced binding affinity.

A monoclonal antibody is an antibody that recognizes a single antigen site. Due to its uniform specificity, a monoclonal antibody is generally more useful than a polyclonal antibody that contains antibodies recognizing many different antigen sites. A monoclonal antibody can be produced by hybridoma methods (Kohler *et al*., Nature 256:495, 1975), recombinant DNA methods (U.S. Patent No. 4,816,567), and so on.

In hybridoma methods, a suitable host animal, such as a mouse, hamster or rhesus monkey, is immunized as described above to produce antibodies that specifically bind to the protein used for immunization, or to induce lymphocytes that can produce the antibodies. Alternatively, a lymphocyte may be immunized *in vitro*. After this, the lymphocyte is fused with a myeloma cell using a suitable fusion agent, such as a polyethylene glycol, to generate hybridomas (Goding, "Monoclonal Antibodies: Principals and Practice", Academic Press, pp.59-103, 1986). Preferably, the produced hybridomas are seeded and cultured on a proper culture media comprising one or more substances that inhibit the proliferation or growth of unfused parental myeloma cells. For example, when a parental myeloma cell lacks the hypoxantin guanine phosphoribosyl transferase enzyme (HGPRT or HPRT), the culture media for the hybridoma typically comprises substances that inhibit the growth of HGRPT deficient cells, i.e., hypoxantin, aminopterin and thymidine (HAT culture media). Preferred myeloma cells include those that can efficiently fuse, that produce antibodies at a stable high level in selected antibody producing cells, and that are sensitive to media such as HAT media. Among the myeloma cell lines preferred myeloma cell lines include mouse myeloma cell lines, such as mouse tumor derived cells MOPC-21 and MPC-11 (obtained from Salk Institute Cell Distribution Center, San Diego, California, USA), and SP-2 and X63-Ag8-653 cells (obtained from the American Type Culture Collection, Rockville, Md. USA). Human myeloma and mouse-human heteromycloma cell lines have also been used for the production of human monoclonal antibodies (Kozbar, J. Immunol. 133:3001, 1984; Brodeur *et al.*, "Monoclonal Antibody Production Techniques and Application", Marcel Dekker Inc, New York, pp.51-63, 1987).

Next, the culture medium in which the hybridomas have been cultured is analyzed for production of monoclonal antibodies against the antigen. Preferably, the binding specificity of a monoclonal antibody produced from a hybridoma cell is measured by an *in vitro* binding assay, such as immunoprecipitation, radioimmunoassay (RIA) or enzyme-linked immunosorbent assay (ELISA) . After identifying the hybridomas that produce antibodies that possess the desired specificity, affinity and/or activity, clones are subcloned by limiting dilution methods and cultured by standard protocols (Goding, "Monoclonal Antibodies: Principals an Practice", Academic Press, pp.59-103, 1986). Culture media suitable for this purpose include, for example, D-MEM and RPMI-1640 media. Furthermore, hybridomas can also be grown *in vivo* as ascites tumors in animals. Monoclonal antibodies secreted from a subclone are preferably purified from culture media, ascites, or serum, via conventional immunoglobulin purification methods, such as protein A-Sepharose, hydroxyapatite chromatography, gel electrophoresis, dialysis or affinity chromatography.

A DNA encoding a monoclonal antibody can be easily isolated and sequenced by conventional methods (such as those using an oligonucleotide probe specifically binding to genes encoding the heavy and light chains of the monoclonal antibody). Hybridomas are preferred starting materials for obtaining such DNAs. Once the DNA is isolated, it is inserted into an expression vector and transformed into a host cell, such as an *E. coli* cell, simian COS cell, Chinese hamster ovary (CHO) cell, or myeloma cell which produces no immunoglobulin unless being transformed, and the monoclonal antibody is produced from the recombinant host cell. In another embodiment, an antibody or an antibody fragment can be isolated from an antibody phage library prepared by a method described by McCafferty *et al.* (Nature 348: 552-554, 1990).

Clackson *et al.* (Nature 352: 624-628, 1991) and Marks *et al.* (J. Mol. Biol. 222: 581-597, 1991) respectively describe the isolation of mouse and human antibodies using phage libraries. The following references describe the production of high affinity (nM range) human antibodies by chain shuffling (Marks *et al,* Bio/Technology 10:779-783, 1992), and combinatorial infection and *in vivo* recombination for producing large phage libraries (Waterhouse *et al,* Nucl. Acids Res. 21:2265-2266, 1993). These techniques can also be used to isolate monoclonal antibodies in place of conventional monoclonal antibody hybridoma techniques.

DNAs encoding monoclonal antibodies can be also altered by, for example, substituting corresponding mouse sequences with the coding sequences of the constant domains of human heavy and light chains (U.S. Patent No. 4,816,567; Morrison *et al*, Proc. Natl. Acad. Sci. USA 81: 6851, 1984), or by binding immunoglobulin polypeptides through covalent bonds. Typically, in these non-immunoglobulin polypeptides, the antibody constant domain or the variable domain of the antibody antigen-binding site is substituted in order to construct a chimeric bispecific antibody that has an antigen-binding site specific for an antigen, and a second antigen-binding site specific for another antigen.

In addition, human antibodies, humanized antibodies, chimeric antibodies, and fragments thereof (for example, Fab, F(ab')₂, and Fv) are also examples of antibodies that bind to the polypeptides of the present invention.

"Humanized antibodies", which are also called "reshaped human antibodies", are prepared by grafting the CDR (complementarity determining region) of a non-human mammalian antibody, for example, a mouse antibody, into the CDR of a human antibody. General methods for recombining humanized antibodies are known in the art. Specifically, humanized antibodies can be obtained by: designing a DNA sequence in which the CDR of a mouse antibody is linked to the framework region (FR) of a human antibody; using PCR to synthesize this DNA sequence using several oligonucleotides that mutually overlap at their terminuses; ligating the DNA thus obtained with a DNA encoding the constant domain of a human antibody; inserting this into an expression vector; and introducing this into host cells for production (see EP 239400 and WO 96/02576). FRs of human antibodies, which are linked through the CDR, may be selected so that the CDR can form a good antigen-binding site. If necessary, amino acids in the FRs of the variable domain of an antibody may be altered so that the reconstituted human antibody CDR can form a proper antigen-binding site (Sato, K. et al., Cancer Res. (1993) 53, 851-856).

The term "chimeric antibodies" means those in which a part of the heavy (H) chain and/or light (L) chain is derived from a specific species or from a specific antibody class or subclass, and the residual part of the chain is derived from other species or from other antibody classes or subclasses.

In the present invention, an "antibody fragment" refers to a part of a full-length antibody, and generally indicates an antigen-binding region or a variable region. For example, antibody fragments include Fab, Fab', F(ab')₂ and Fv fragments. Papain digestion of an antibody produces two identical antigen-binding fragments called Fab fragments, each comprising an antigen-binding region, and a remaining fragment called "Fc" because it crystallizes easily. On the other hand, pepsin digestion results in an F(ab')₂ fragment (which has two antigen-binding sites and can cross bind antigens) and the other remaining fragment (called pFc'). Other fragments include diabodies, linear antibodies, single-chain antibodies, and multispecific antibodies formed from antibody fragments.

Herein, an "Fv" fragment is the smallest antibody fragment and contains a complete antigen recognition site and a binding site. This region is a dimer (a V_{H}-V_{L} dimer) wherein the variable domains of each of the heavy chain and light chain are strongly connected by a noncovalent bond. The three CDRs of each of the variable domains interact with each other to form an antigen-binding site on the surface of the V_{H}-V_{L} dimer. Six CDRs confer the antigen-binding site of an antibody. However, even a single variable domain (or half of an Fv which comprises only three antigen-specific CDRs) also comprises the ability to recognize and bind an antigen, although it has a lower affinity than that of the complete binding site.

Moreover, a Fab fragment (also referred to as F(ab)) further includes the constant domain of a light chain and a cellular constant domain (C_{H}l) of a heavy chain. A Fab' fragment differs from the Fab fragment in that it has a few extra residues derived from the carboxyl end of the heavy chain C_{H}l domain, which comprises one or more cysteines from the hinge domain of the antibody.

In the present invention, "diabody (diabodies)" refers to small antibody fragments comprising two antigen-binding sites. The fragments comprise V_{H}-V_{L} wherein a heavy chain variable domain (V_{H}) is connected to a light chain variable domain (V_{L}) in the same polypeptide chain. When a short linker is used between the two domains so that the two regions cannot be connected together in the same chain, these two domains form pairs with the constant domains of another chain, creating two antigen-binding sites. Diabodies are described in detail in, for example, European Patent No. 404,097, WO 93/11161 and Holliger P. *et al.* (Proc. Natl. Acad. Sci. USA 90:6444-6448, 1993) .

A single-chain antibody (hereafter also referred to as a single-chain Fv or sFv) or sFv antibody fragment comprises the V_{H} and V_{L} domains of an antibody, and these domains exist on a single polypeptide chain. Generally, an Fv polypeptide further contains a polypeptide linker between the V_{H} and V_{L} domains, and therefore an sFv can form a structure necessary for antigen binding. For a review of sFv, refer to Pluckthun "The Pharmacology of Monoclonal Antibodies" Vol.113 (Rosenburg and Moore eds. (Springer Verlag, New York) pp.269-315, 1994).

A multispecific antibody is an antibody specific to at least two different kinds of antigen. Although such a molecule usually binds to two antigens (i.e., a bispecific antibody), a "multispecific antibody" herein encompasses antibodies with specificity to more than two antigens (for example, three antigens). The multispecific antibodies can be full-length antibodies or fragments thereof (for example, a F(ab')₂ bispecific antibody).

Conventionally, antibody fragments have been produced by protease digestion of natural antibodies (Morimoto *et al*., J. Biochem. Biophys. Methods 24:107-117, 1992; Brennan *et al*., Science 229:81, 1985), but nowadays they can also be produced by recombinant techniques. For example, antibody fragments can also be isolated from the above-mentioned antibody phage library. Furthermore, F(ab')₂-SH fragments can be directly collected from host cells such as *E. coli,* and chemically bound in the form of F(ab')₂ fragments (Carter, et al., Bio/Technology 10:163-167, 1992). Moreover, in another method, F(ab')₂ fragments can also be directly isolated from recombinant host cell cultures. Methods for preparing fragments of single chain antibodies and such are well known. Methods for constructing single chain antibodies are well known in the art (for example, see, U.S. Patent No. 4,946,778; U:S. Patent No. 5,260,203; U.S. Patent No. 5,091,513; U.S. Patent No. 5,455,030; etc.).

Methods for producing multispecific antibodies are known in the art. The production of full-length bispecific antibodies comprises the step of co-expressing two immunoglobulin heavy-light chains with different specificities (Millstein *et al*., Nature 305:537-539, 1983). The heavy and light immunoglobulin chains are randomly combined, and the obtained multiple co-expressing hybridomas (quadroma) are therefore a mixture of hybridomas, each expressing a different antibody molecule. Thus, hybridomas producing the correct bispecific antibody must be selected among them. The selection can be performed by methods such as affinity chromatography. Furthermore, according to another method, the variable domain of an antibody comprising the desired binding specificity is fused to the constant domain sequence of an immunoglobulin. The above-mentioned constant domain sequence preferably comprises at least a part of the hinge, CH2 and the CH3 regions of the heavy chain constant domain of the immunoglobulin. Preferably, the CH1 region of the heavy chain, required for binding with the light chain, is also included. A DNA encoding the immunoglobulin heavy chain fusion is inserted into an expression vector to transform a proper host organism. If needed, a DNA encoding the immunoglobulin light chain is also inserted into an expression vector different from that of the immunoglobulin heavy chain fusion, to transform the host organism. There are cases where the antibody yield increases when the ratio of the chains is not identical. In such cases, inserting each of the genes into separate vectors is more convenient, since the expression ratio of each of the chains can be controlled. However, genes encoding multiple chains can also be inserted into a vector.

According to a preferred embodiment, a bispecific antibody is desired wherein a heavy chain comprising a first binding specificity exists as an arm of a hybrid immunoglobulin, and a heavy chain-light chain complex comprising another binding specificity exists as the other arm. The bispecific antibody can be readily isolated from other immunoglobulins since the light chain exists on only one of the arms. Such separation methods are referred to in WO 94/04690. For further reference to methods for producing bispecific antibodies, see Suresh *et al.* (Methods in Enzymology 121:210, 1986). As methods for reducing homodimers to increase the ratio of heterodimers in the final product obtained from recombinant cell culture, methods are also known wherein a pocket corresponding to a bulky side chain of a first antibody molecule is created in a multispecific antibody that comprises the antibody constant domain CH3 (WO 96/27011). In this method, one or more light-chain amino acids, which are on the surface of one of the antibody molecules and bind to the other molecule, are changed to amino acids with a bulky side chain (e.g., tyrosine or tryptophan). Furthermore, amino acids with a bulky side chain in the corresponding portion of the other antibody molecule are changed to amino acids with a small side chain (e.g., alanine or threonine).

Bispecific antibodies include, for example, heteroconjugated antibodies wherein one antibody is bound to avidin and the other to biotin and such (U.S. Patent No. 4,6.76,980, WO 91/00360, WO 92/00373, European Patent No. 03089). Cross-linkers used for the production of such heteroconjugated antibodies are well known, and are mentioned, for example in U.S. Patent No. 4, 676, 980.

Additionally, methods for producing bispecific antibodies from antibody fragments have been also reported. For example, bispecific antibodies can be produced by utilizing chemical bonds. For example, first, F(ab')₂ fragments are produced and the fragments are reduced in the presence of the dithiol complexing agent, sodium arsanilate, to prevent intramolecular disulfide formation. Next, the F(ab')₂ fragments are converted to thionitrobenzoate (TNB) derivatives. After using mercaptoethanolamine to again reduce one of the F(ab')₂-TNB derivatives to a Fab'-thiol, equivalent amounts of the F(ab')₂-TNB derivative and Fab'-thiol are mixed to produce a bispecific antibody.

Various methods have been reported for directly producing and isolating bispecific antibodies fromrecombinant cell cultures. For example, a production method for bispecific antibodies using a leucine zipper has been reported (Kostelny *et al*., J. Immunol. 148:1547-1553, 1992). First, leucine zipper peptides of Fos and Jun proteins are conjugated to the Fab' sites of different antibodies by gene fusion. The homodimer antibodies are reduced at the hinge region to form monomers, and then reoxidized to form heterodimer antibodies. Alternatively, there are methods for forming two antigen-binding sites, wherein pairs are formed between different complementary light chain variable domains (VL) and heavy chain variable domains (VH) by linking the VL and VH domains through a linker that is short enough to prevent pairing of these two domains (Hollinger *et al*., Proc. Natl. Acad. Sci. USA 90:6444-6448, 1993). Furthermore, dimers utilizing a single chain Fv (sFV) have also been reported (Gruger *et al.*, J. Immunol. 152:5368, 1994). Moreover, trispecific (rather than bispecific) antibodies have also been reported (Tutt *et al.*, J. Immunol. 147:60, 1991).

Human antibodies can be obtained by methods known to those skilled 'in the art. For example, subject human. antibodies can be obtained by (a) sensitizing human lymphocytes *in vitro*; and (b) fusing the sensitized lymphocytes thus obtained with human-derived myeloma cells capable of differentiating permanently (Examined Published Japanese Patent Application No. (JP-B) Hei 1-59878). Alternatively, antibody-producing cells can be generated by administering antigens to transgenic animals that comprise a full repertoire of human antibody genes, and then immortalizing them to obtain human antibodies (see WO 94/25585, WO 93/12227, WO 92/03918, and WO 94/02602).

By using recombinant techniques, recombinant hosts can be produced by transforming hosts with cDNAs that encode each of the heavy and light chains of such humanized antibodies, preferably using vectors that contain the cDNAs. Hosts that produce recombinant human monoclonal antibodies are then cultured, and the humanized antibodies are obtained from the supernatant. Herein, such hosts are eukaryotic cells other than fertilized eggs, preferably mammalian cells, such as CHO cells, lymphocytes and myeloma cells.

The resulting antibodies may be uniformly purified. General methods for isolating and purifying proteins may be used to isolate and purify antibodies. Isolation and purification of antibodies can be accomplished by selecting or appropriately combining, for example, column chromatography comprising affinity chromatography, filtration, ultrafiltration, salting out processes, dialysis, SDS-polyacrylamide gel electrophoresis, and isoelectric focusing (Antibodies: A Laboratory Manual. Ed Harlow and David Lane, Cold Spring Harbor Laboratory, 1988), but these examples are not limiting. Columns for affinity chromatography include Protein A columns and Protein G columns. For example, Hyper D, POROS, and Sepharose F. F. (Pharmacia) may be used as Protein A columns.

Furthermore, antibodies may be bound to various reagents for therapeutic use. Such reagents comprise chemotherapeutic agents such as doxorubicin, methotrexate, and taxol, heavy metals, radioactive nuclides, and toxins such as Pseudomonas antidotes. Methods for producing therapeutic reagent-antibody conjugates, and their therapeutic applications, are described in US 5057313 and US 5156840.

The present invention also provides oligonucleotides comprising at least 15 nucleotides that are complementary to a DNA of the present invention, or to a complementary strand thereof.

A "complementary strand" herein refers to one strand of a double strand DNA comprising A:T (or A:U for RNA) and G:C base pairs, when viewed against the other strand. Furthermore, "complementary" means not only nucleotide sequences completely complementary to a continuous nucleotide sequence of at least 15 nucleotides, but also those with a homology of at least 70%, preferably at least 80%, more preferably 90%, and most preferably 95% or more at the nucleotide sequence level. Homology can be determined using an algorithm described herein. The term "oligonucleotide" also comprises polynucleotides.

The oligonucleotides of the present invention can be used as probes and primers for the detection and amplification of DNAs encoding the polypeptides of the present invention; probes and primers for detecting the expression of the DNAs; and nucleotides and nucleotide derivatives (for example, antisense oligonucleotides or DNA encoding ribozymes, or DNAs encoding them) used for controlling the expression of a polypeptide of the present invention. Moreover, the oligonucleotides of the present invention may be used in the form of a DNA-array substrate.

When used as primers, the oligonucleotides may usually comprise 15 to 100 bp, preferably 17 to 30 bp. The primer is not limited, as long as it can amplify at least a portion of a DNA of the present invention, or a complementary strand thereof. In addition, when used as a primer, the 3' side of the oligonucleotide may be designed to be complementary, and a restriction enzyme recognition sequence or tag may be attached to the 5' side of the same.

Moreover, when an above-described oligonucleotide is used as a probe, the probe is not particularly limited as long as it hybridizes specifically to at least a portion of a DNA of the present invention, or a complementary strand thereof. The probe may be also a synthetic oligonucleotide, which usually comprises at least 15 bp or more.

When the oligonucleotides of the present invention are used as probes, they are preferably appropriately labeled. Examples of labeling methods comprise methods in which T4 polynucleotide kinase is used to label the 5' end of an oligonucleotide by ³²P phosphorylation or methods in which DNA polymerases such as Klenow enzyme, and primers such as random hexamer oligonucleotides, are used to incorporate nucleotides labeled with isotopes such as ³²P, fluorescent dyes, biotin, and such ( random priming methods and the like) into the oligonucleotide substrate.

The oligonucleotides of the present invention may be produced by, for example, a commercially available oligonucleotide synthesizer Probes may also be prepared as double-stranded DNA fragments by restriction enzyme treatment and such.

Antisense oligonucleotides comprise, for example, those which hybridize to any portion of the nucleotide sequence of SEQ ID NO: 1. The antisense oligonucleotides are preferably antisense to a nucleotide sequence comprising at least 15 consecutive nucleotides or more of SEQ ID NO: 1. More preferably, the nucleotide sequences comprising at least 15 consecutive nucleotides or more comprise a translational initiation codon.

Derivatives of antisense oligonucleotides, or modified antisense oligonucleotides may also be used. The modified antisense oligonucleotides comprise antisense oligonucleotides that are modified by, for example, lower alkylphosphonates such as methylphosphonates and ethylphosphonates, phosphorothioates, or phosphoroamidates.

The antisense oligonucleotides are not restricted to those in which all nucleotides are complementary to the corresponding nucleotides making up a given region of DNA or mRNA. So long as an oligonucleotide can specifically hybridize with a DNA comprising the nucleotide sequence of SEQ ID NO: 1, or a mRNA encoded thereby, it may have one or more nucleotide mismatches.

The derivatives of the antisense oligonucleotides of the present invention may inhibit the function of the polypeptides of the present invention by acting on cells that produce the polypeptides of the present invention. By binding to DNAs or mRNAs that encode the polypeptides, their transcription or translation may be inhibited or the degradation of mRNAs may be promoted, and thus expression of the polypeptides of the present invention is suppressed.

The derivatives of the antisense oligonucleotides of the present invention may be mixed with an appropriate base that is inert against the derivative, and used as a medicine for external applications, such as salves or poultices.

If necessary, the derivatives may be mixed with excipients, isotonizing agents, solubilizing agents, stabilizers, preservatives analgesic agents, or such to be prepared as a tablet, powder, granule, capsule, liposome capsule, injectable solution, liquid formulation, nose drop, freeze-dried agent, or such. They may be prepared according to standard methods.

The derivatives of the antisense oligonucleotides of the present invention may be, for example, directly applied to the lesional area of a patient, or administered into blood vessels so as to eventually reach the lesional area. Moreover, the derivatives may be encapsulated in antisense-encapsulating materials such as liposomes, poly-L-lysine, lipids, cholesterol, lipofectin, or their derivatives, in order to increase durability and/or membrane permeability.

Doses of the derivatives of the antisense oligonucleotides of the present invention may be appropriately adjusted depending on the patient's condition condition, and may preferably be administered in an amount in the range of, for example, 0.1 to 100 mg/kg, and preferably 0.1 to 50 mg/kg.

Since the antisense oligonucleotides of the present invention inhibit the expression of the polypeptides of the present invention, they are useful in suppressing the biological activities of the polypeptides of the present invention. Also, expression inhibitors comprising the antisense oligonucleotides of the present invention are useful in that they can suppress the biological activities of the polypeptides of the present invention.

According to the present invention, the term "substrate" refers to a plate material that can immobilize oligonucleotides. There are no limitations as to the substrates of the present invention so long as oligonucleotides can be immobilized on the substrate, but a substrate generally used for DNA array technologies may be preferably used.

Generally, DNA arrays consist of several thousands of oligonucleotides printed on a substrate at a high density. Usually, these DNAs are printed onto the surface of a non-porous substrate. The substrate surface is usually made of glass, but a porous membrane, for example, a nitrocellulose membrane may be used.

According to the present invention, methods for immobilizing (arraying) oligonucleotides are, for example, the oligonucleotide-based array technology developed by Affymetrix Inc. In oligonucleotide arrays, the oligonucleotides are usually synthesized in situ. For example, photolithographic technology (Affymetrix Inc.), and inkjet technology for immobilizing chemical substances (Rosetta Inpharmatics Inc.), are already known as methods for in situ oligonucleotide synthesis, both of which can be used to prepare the substrate of the present invention.

When oligonucleotides are immobilized to the substrate in the present invention, the nucleotide probes bound to the substrate usually consist of 10 to 100, preferably 10 to 50, and more preferably 15 to 25 nucleotides.

Furthermore, this invention provides methods of screening for compounds that bind to the polypeptides of the present invention. These methods comprise the steps of: contacting a test sample that is expected to comprise a compound that binds to a polypeptide of the present invention with the polypeptides of the present invention; detecting the binding activity of the polypeptide to the test sample; and selecting compounds that comprise the activity of binding to the polypeptide of the present invention.

The polypeptides of the invention used for screening may be recombinant or natural polypeptides, or partial peptides. Alternatively, they may be in a form expressed on the surface of a cell, or in the form of a membrane fraction. Samples tested include, but are not limited to, cell extracts, cell culture supernatants, products of fermentation microorganisms, marine organism extracts, plant extracts, purified or crude preparations of polypeptides, non-peptide compounds, synthetic low-molecular weight compounds, and natural compounds. For example, the polypeptides of the present invention to be contacted with a test sample may be brought into contact with the test sample as a purified polypeptide, as a soluble polypeptide, in a form attached to a carrier, as a fusion polypeptide with other polypeptides, in a form expressed on a cell membrane, or as a membrane fraction.

Various methods known to those skilled in the art may be used as methods of screening for polypeptides that bind to the polypeptides of the present invention using a protein of the present invention. Such screening can be carried out, for example, by immunoprecipitation methods. Specifically, the methods can be carried out as follows: DNAs encoding the polypeptides of this invention are expressed by inserting them into vectors for foreign gene expression, such as pSV2neo, pcDNA I, and pCD8; and then expressing the gene in animal cells, etc. Any generally used promoter may be employed for the expression, including the SV40 early promoter (Rigby In Williamson (ed.), Genetic Engineering, Vol. 3. Academic Press, London, p.83-141 (1982)), EF-1α promoter (Kim, et al. Gene 91, p. 217-223 (1990)), CAG promoter (Niwa, et al. Gene 108, p. 193-200 (1991)), RSV LTR promoter (Cullen, Methods in Enzymology 152, p.684-704 (1987)), SR α promoter (Takebe et al., Mol. Cell. Biol. 8, p.466 (1988)), CMV immediate early promoter (Seed and Aruffo Proc. Natl. Acad. Sci. USA 84, p. 3365-3369 (1987)), SV40 late promoter (Gheysen and Fiers J. Mol. Appl. Genet. 1, p.385-394 (1982)), Adenovirus late promoter (Kaufman et al., Mol. Cell. Biol. 9, p.946 (1989) ), HSV TK promoter, etc.

Introduction of a foreign gene into animal cells for expression therein can be performed by any of the following methods, including electroporation methods (Chu, G. et al., Nucl. Acid Res. 15, 1311-1326 (1987)), calcium phosphate methods (Chen, C. and Okayama, H. Mol. Cell. Biol. 7, 2745-2752 (1987)), DEAE dextran methods (Lopata, M. A. et al. Nucl. Acids Res. 12, 5707-5717 (1984); Sussman, D. J. and Milman, G. Mol. Cell. Biol. 4, 1642-1643 (1985)), lipofectin methods (Derijard, B. Cell. 7, 1025-1037 (1994); Lamb, B. T. et al. Nature Genetics 5, 22-30 (1993)), Rabindran, S. K. et al. Science 259, 230-234 (1993)), etc.

The polypeptides of this invention can be expressed as fusion polypeptides that comprise a recognition site of a monoclonal antibody whose specificity has been established, by introducing the recognition site (epitope) into the N- or C-terminus of the polypeptides of this invention. For this purpose, a commercial epitope-antibody system can be utilized (Jikken Igaku (Experimental Medicine) 13, 85-90 (1995)). Vectors that express fusion polypeptides via a multi-cloning site with β-galactosidase, maltose-binding protein, glutathione S-transferase, green fluorescence protein (GFP), and such are commercially available.

To minimize alterations in the properties of the polypeptides of this invention, which may be caused by fusion polypeptide formation, methods for preparing fusion polypeptides have been reported that involve introducing only a small epitope portion, comprising a few to dozens of amino acid residues. For example, the epitopes of polyhistidine (His-tag), influenza hemagglutinin (HA), human c-myc, FLAG, Vesicular stomatitis virus glycoprotein (VSV-GP), T7 gene 10 protein (T7-tag), human herpes simplex virus glycoprotein (HSV-tag), E-tag (epitope on the monoclonal phage), and such, and monoclonal antibodies that recognize these epitopes, can be utilized as epitope-antibody systems for screening polypeptides that bind to the polypeptides of this invention (Jikken Igaku (Experimental Medicine) 13, 85-90 (1995)).

For immunoprecipitation, immune complexes are formed by adding these antibodies to cell lysates prepared using suitable surfactants. These immune complexes comprise a polypeptide of this invention, a polypeptide that binds to this polypeptide, and an antibody. In addition to using antibodies against the above-described epitopes to perform immunoprecipitation, antibodies against the polypeptides of this invention can also be used. An antibody against a polypeptide of this invention can be prepared by, for example, inserting a gene encoding a polypeptide of this invention into an appropriate expression vector of *E. coli*, purifying the polypeptide thus expressed in the bacterium, and immunizing rabbits, mice, rats, goats, chickens, and such, with the purified protein. The antibodies can also be prepared by immunizing the above-described animals with partial peptides of the polypeptides of this invention.

When the antibody is a murine IgG antibody, immune complexes can be precipitated using, for example, Protein A Sepharose and Protein G Sepharose. In addition, when the polypeptides of this invention are prepared as fusion polypeptides with the epitope of, for example, GST or such, the immune complexes can be formed using substances that specifically bind to these epitopes, such as glutathione-Sepharose 4B, giving the same result as when using an antibody for a polypeptide of this invention.

In general, immunoprecipitation may be carried out according to, or in line with, the methods described in the literature (Harlow, E. and Lane, D. : Antibodies, pp. 511-552, Cold Spring Harbor Laboratory publications, New York (1988)).

SDS-PAGE is generally used to analyze immunoprecipitated polypeptides. By using a gel of an appropriate concentration, bound polypeptides can be analyzed based on their molecular weight. Generally, in such cases the polypeptides that have bound to the polypeptides of this invention can hardly be detected by usual polypeptide staining methods, such as Coomassie staining and silver staining. However, detection sensitivity can be improved by culturing the cells in a medium comprising radio isotope-labeled ³⁵S-methionine and ³⁵S-cysteine to label polypeptides inside the cells, and detecting the labeled polypeptides. After determining the molecular weight of a desired polypeptide, the polypeptide can be directly purified from an SDS-polyacrylamide gel, and then sequenced.

Polypeptides that bind to the polypeptides of the present invention by using these polypeptides may be isolated by West-Western blotting (Skonik E.Y. et al. (1991) Cell 65: 83-90). Specifically, by using phage vectors (λgt11, ZAP, etc.), a cDNA library is constructed from cells or tissues that are expected to express polypeptides that bind to a polypeptide of the present invention. This cDNA library is then expressed on an LB-agarose plate and transferred to a filter membrane. The filter membrane is reacted with purified and labeled polypeptides of the invention. Plaques expressing polypeptides that bind to the polypeptide of the invention can be identified by detecting the label. The polypeptides of the invention may be labeled by methods that utilize binding between biotin and avidin, or methods utilizing antibodies that specifically bind to a polypeptide of the present invention, or a polypeptide (such as GST) that is fused to a polypeptide of the present invention. Methods using radioisotopes or fluorescence and such may also be used.

Alternatively, according to another embodiment of the screening methods of the present invention, a two-hybrid system that utilizes cells may be used (Fields S. and Sternglanz R. (1994) Trends Genet. 10: 286-292; Dalton S. and Treisman R. (1992) "Characterization of SAP-1, a protein recruited by serum response factor to the c-fos serum response element." Cell 68: 597-612; "MATCHMAKER Two-Hybrid System", "Mammalian MATCHMAKER Two-Hybrid Assay Kit", "MATCHMAKER One-Hybrid System" (products of Clontech); "HybriZAP Two-Hybrid Vector System" (Stratagene)).

A two-hybrid system can be used as follows: (1) a polypeptide of the present invention or partial peptide thereof is fused to a SRF DNA binding region or GAL4 DNA binding region, and expressed in yeast cells; (2) a cDNA library, which expresses polypeptides as fusion polypeptides with VP16 or GAL4 transcription activating regions, is prepared from cells expected to express the polypeptides binding to the polypeptide of the present invention; (3) the library is introduced to the above-mentioned yeast cells; and (4) library-derived cDNAs are isolated from the positive clones detected (positive clones can be confirmed by the activation of reporter genes on binding of the present polypeptide and the binding polypeptide expressed in the yeast cell). The polypeptides encoded by the cDNAs can be obtained by introducing and expressing the isolated cDNAs in *E. coli.* Thus, polypeptides that bind to the present polypeptides, or genes thereof, can be prepared.

In addition to the HIS3 gene, reporter genes for use in two-hybrid systems include, for example, the Ade2 gene, LacZ gene, CAT gene, luciferase gene, PAI-1 (Plasminogen activator inhibitor type1) gene, and such, but are not restricted thereto. In addition to yeast, screening using a two-hybrid system can be also carried out using mammalian cells.

Alternatively, compounds binding to the polypeptides of the present invention can be screened using affinity chromatography. For example, the polypeptides of the invention are immobilized to an affinity column carrier, and a test sample predicted to express polypeptides that bind to a polypeptide of the invention is applied to the column. The test samples used herein may be cell extracts, cell lysates, and so on. After loading the test samples, the column is washed, and polypeptides that bind to the polypeptides of the invention can be obtained.

The DNAs that encode the polypeptides may be obtained by analyzing the amino acid sequence of the obtained polypeptides, synthesizing oligo-DNAs based on this sequence information, and screening a cDNA library using these DNAs as probes.

In addition, methods for isolating not only polypeptides, but also compounds (including agonists and antagonists) that bind to the polypeptides of the invention are known in the art. Such methods include, for example, methods of screening for molecules that bind to the polypeptides by contacting synthesized compounds or natural substance banks, or random phage peptide display libraries, with immobilized polypeptides of the invention; as well as high-throughput screening methods that use combinatorial chemistry techniques (Wrighton, N. C., Farrell, F. X., Chang R., Kashyap A. K., Barbone F. P., Mulcahy L. S., Johnson D. L., Barrett R. W., Jolliffe L. K., and Dower W. J., "Small peptides as potent mimetics of the protein hormone erythropoietin" Science (UNITED STATES), Jul 26, 1996, 273 p458-64; Verdine G.L., "The combinatorial chemistry of nature" Nature (ENGLAND), Nov 7, 1996, 384, p11-13; Hogan J. C. Jr., "Directed combinatorial chemistry" Nature (ENGLAND) Nov 7, 1996, 384, p17-9).

Biosensors utilizing the phenomenon of surface plasmon resonance may be used to detect or measure a compound bound in the present invention. When such biosensors are used, interactions between a polypeptide of the invention and a test compound can be observed in real-time, as surface plasmon resonance signals use only a small amount of polypeptides without labeling (for example, BIAcore, Pharmacia). Therefore, binding between a polypeptide of the invention and a test compound can be evaluated using a biosensor like BIAcore.

Compounds that can be isolated by the screening of the present invention may serve as candidate drugs to regulate the activity of a polypeptide of the invention, and thus can be applied to the treatment of diseases caused by abnormal expression or function or the like of the polypeptides, and diseases capable of being treated by regulating the activity of a polypeptide of the invention. Target diseases for therapy and prevention are preferably inflammatory skin diseases. This invention also encompasses compounds that can be isolated by such screening.

This invention provides methods of testing for inflammatory skin diseases, where the methods comprise the step of determining the amount of SLURP-2 gene expression. Specific embodiments of the test methods of the present invention are described below, but the invention is not limited thereto.

One embodiment of the testing methods comprises the steps of: preparing an RNA sample from a test subject; determining the amount of RNA that encodes a polypeptide of the present invention in the RNA sample; and finally comparing the determined amount of RNA with that from a control sample. Another embodiment comprises the steps of: preparing a cDNA sample from a test subject; determining the amount of cDNA encoding a polypeptide of the present invention in the cDNA sample; and finally comparing the determined amount of cDNA with that of a control sample. These methods comprise techniques known to those skilled in the art, for example, Northern blotting, RT-PCR, and DNA array methods.

DNA array methods comprise the steps of: preparing cDNA samples by using RNAs prepared from test subjects as templates; contacting the samples with a substrate to which oligonucleotides of the present invention are immobilized; detecting the hybridization strength of the cDNA samples to the oligonucleotide probes immobilized on the substrate, to thus determine the amount of SLURP-2 gene expressed in the cDNA samples; and finally, comparing the expressed amount of SLURP-2 gene thus determined with that of a control sample.

Methods for preparing a cDNA sample from a test subject are known to those skilled in the art. In a preferred embodiment for preparing cDNA samples, total RNAs may be first prepared from cells (such as skin keratinocytes) or tissues by methods known to those skilled in the art, for example, as below. Any known methods and kits may be used for preparing the total RNA, as long as highly pure total RNA can be prepared. For example, after pretreating a sample using "RNA later" from Ambion Inc., "Isogen" from Nippon Gene Co. Ltd. is used to prepare the total RNA. These methods can be carried out according to their attached protocols.

The extracted total RNA may be used as a template for synthesizing cDNAs, using reverse transcriptase to prepare cDNA samples. Methods known to those skilled in the art can be used to synthesize cDNAs from total RNA. Optionally, the cDNA samples thus prepared can be labeled for detection. The labeling substances are not limited, so long as they can be detected, and are, for example, fluorescent substances and radioisotopes. Labeling can be carried out according to methods commonly used in the art (L Luo et al., Gene expression profiles of laser-captured adjacent neuronal subtypes. Nat. Med. 1999, 117-122).

The hybridization strength of a cDNA to a nucleotide probe can be appropriately detected by those skilled in the art, in line with the type of the substance used to label the cDNA sample. For example, when the cDNA is labeled with a fluorescent substance, strength can be detected by using a scanner to read the fluorescence signal.

In another embodiment, a testing method in the present invention comprises the steps of: preparing a polypeptide sample from a test subject's cells (for example, skin keratinocytes) or tissues ; determining the amount of the polypeptide of the present invention comprised in the polypeptide sample; and comparing the amount of the polypeptide with that in the control sample.

Such methods comprise SDS-polyacrylamide gel electrophoresis, as well as Western blotting, dot blotting, immunoprecipitation, enzyme-linked immunosorbent assays (ELISA), and immunofluorescent methods, which use the antibodies of the present invention.

In the methods described above, if the expression levels of SLURP-2 gene are significantly increased compared with a control, then the subject is suspected to be affected with (at high risk of) an inflammatory skin disease in the future, or is determined to be infected with the disease.

This invention also provides test agents for the methods for testing inflammatory skin diseases. Examples of such test agents are test agents comprising an oligonucleotide of the present invention (comprising a substrate on which an oligonucleotide probe is immobilized), and a test agent comprising an antibody of the present invention. The above-mentioned antibody is not limited, as long as it can be used for testing. The antibodies may be labeled, as necessary.

In addition to the oligonucleotides or the antibodies that are active ingredients, the above-mentioned test agents may be optionally mixed with, for example, sterile water, saline, plant oils, detergents, lipids, solubilizing agents, buffers, protein stabilizers (such as BSA and gelatin), and preservatives.

### Brief Description of the Drawings

Fig. 1 is a map showing the nucleotide sequence of SLURP-2 cDNA, the deduced amino acid sequence, and the nucleotide sequence of the promoter regions. Arrows indicate transcriptional initiation sites. The figure lists the nucleotide sequence and amino acid sequence as SEQ ID NOs: 1 and 2, respectively.
Fig. 2 is an alignment of the amino acid sequences of Ly-6 superfamily members in humans and mice. Conserved cysteine residues are boxed in solid lines. The amino acid sequences of SLURP-1, E48, Ly61 (mouse), and RIG-E are listed as SEQ ID NOs: 14-17, respectively.
Fig. 3 is a photograph showing tissue expression analysis by RT-PCR. The SLURP-2 gene is mainly expressed in epithelial tissues, with its highest expression in the esophagus and uterine cervix, followed by skin and keratinocytes. Expression is observed in the thymus, but not in the bone marrow or spleen. GAPDH was also measured as a loading control.
Fig. 4 is a photograph showing Northern hybridization analysis of SLURP-2 mRNA expression. SLURP-2 mRNA is expressed in the esophagus, stomach, and duodenum, although its length differs. The tissues examined were: lane 2, esophagus; lane 3, stomach; lane 4, duodenum; lane 5, ileocecum; lane 6, ileum; lane 7, jejunum; lane 8, ascending colon; lane 9, descending colon; lane 10, transverse colon; lane 11, rectum; lane 12, appendix; and lane 13, liver. A β-actin probe was used as a control.
Fig. 5 is a photograph showing genomic Southern blotting analysis. Ten micrograms of human genome DNA was digested with KpnI, EcoRI + BgIII, and KpnI + BamHI, followed by separation on a 0.6% agarose gel. A fragment corresponding to the exon 3 region was used to search a blot probe.
Fig. 6 shows SLURP-2 mRNA expression in psoriatic lesional skin, lesion-free skin, and normal skin, using quantitative real-time RT-PCR. The results are shown as a mean ±SD (n= 5 for L and NL; n= 4 for N) . Asterisks indicate statistically significant values where P<0.0001.
Fig. 7 presents a physical map and transcript map of human chromosome 8q24.3.

### Best Mode for Carrying out the Invention

The present invention will be specifically illustrated below with reference to Examples, but it is not to be construed as being limited thereto.

### (1) Sample Information

Skin biopsy specimens were removed from the lesional and non-lesional skin of patients, and from normal controls. In all cases, the presence or absence of psoriasis was assessed by a patient's medical history and clinical evaluation. Informed consent was obtained from all individuals from whom skin biopsies were taken. The protocol for removing biopsy specimens from these patients was approved by the Tokai University School of Medicine. All patients were Japanese.

### (2) Microarray Experiments (GeneChip Expression Analysis)

The GeneChip Human Genome probe arrays, U95A, U95B, U95C, U95D, and U95E (Affymetrix, Santa Clara, CA), which contain a set of oligonucleotide probes corresponding to a total of approximately 60,000 genes, were used for gene expression screening according to the manufacturer's protocol (Expression Analysis Technical Manual) . Total RNA was purified from individual skin biopsy specimens using the RNaqueous Reagent (Ambion), according to the manufacturer's protocol. The RNA was treated with DNaseI to remove contaminating genomic DNA, and then precipitated using ethanol. The quality of total RNA was evaluated by agarose gel electrophoresis (degradation of the 28S and 18S bands may not be visible with the naked eye), and by a spectrophotometric method. Double-stranded cDNAs were then synthesized using the Superscript Choice system (Life Technologies, Rockville MD) and a T7 (dT) 24 primer (GENSET). *In vitro* transcription of the cDNAs was carried out in the presence of a biotinylated nucleotide triphosphate, using the BioArray HighYield RNA Transcript Labeling Kit (Enzo Diagnostics, Farmingdale, NY). This biotinylated cRNA was hybridized with U95A-U95E arrays at 45°C for 16 hours. After washing, hybridized biotinylated cRNAs were stained with streptavidin-phycoerythrin (Molecular Probes, Eugene, OR), and scanned using the HP Gene Array Scanner. The fluorescent intensity of the probe was quantified using a computer program GeneChip Analysis Suite 3.3 (Affymetrix).

### (3) Homology Search

ESTs were selected based on the microarray analysis data of cDNAs prepared from the skin of patients with psoriasis vulgaris and the skin of healthy subjects, and their novelty was verified by a homology search using the BLAST algorithm (http://www.ncbi.nlm.nih.gov/BLAST). One of the novel ESTs (GenBank accession No: AI829641) was cloned.

### (4) Cloning a full-length cDNA

Total RNA was extracted from cultured keratinocytes using TRIzol Reagent (Gibco BRL, Gaithersburg, MD), and mRNA was purified from the extracts using a Dynabeads mRNA Purification kit (VERITAS). RACE (rapid amplification of the 5'- and 3'-terminal cDNA) libraries were constructed using Marathon and SMART RACE cDNA Amplification Kit protocols (Clontech, Palo Alto, CA). To obtain the 3' and 5' termini of this cDNA, gene-specific primers, 5'-CTAGGACAAGCGGTGCTGGACGG-3' (SEQ ID NO: 3) for the 3'-RACE library and 5'-CTAGGACAAGCGGTGCTGGACGG-3' (SEQ ID NO: 4) for the 5'-RACE library, and their universal adaptor primers (AP-1) (Clontech) were used in PCR. After purification, the PCR products were cloned into pGEM-T easy vectors (Promega) . Twenty clones were sequenced on both strands using an ABI PRISM 3700 DNA Analyzer (Applied Biosystems). The 5' end of this cDNA was confirmed by oligo-capping. The 5'-RLM-RACE protocol of the FirstChoice RLM-RACE (Ambion) was used to prepare an oligo-capped cDNA library from cultured keratinocytes. 3 µl of cDNA was amplified using gene-specific primers, 5'-TAGGACAAGCGGTGCTGGACG-3' (SEQ ID NO: 5) and 5'-GGCAGCAAGCGATGGATACGTAG-3' (SEQ ID NO: 6), and 5' RACE Outer primers by touchdown PCR under the following conditions: five cycles of five seconds at 96°C and four minutes at 69°C; ten minutes at 95°C; five cycles of five seconds at 96°C, ten seconds at 67°C, and four minutes at 72°C; 30 cycles of five seconds at 96°C, ten seconds at 67°C, and four minutes at 72°C; and seven minutes at 72°C. The Outer PCR products were then amplified using gene-specific primers, 5'-GGCAGCAAGCGATGGATACGTAG-3' (SEQ ID NO: 7) and 5' -CAGTGCCGAGCTGCATGTTC-3' (SEQ ID NO: 8), and 5' RACE Inner primers under similar cycling conditions, except that the annealing temperature in each cycle was increased by 2°C. After agarose gel electrophoresis and Et-Br staining, the cDNA among the PCR fragments was extracted from the agarose gel and purified. Individual cDNAs were then ligated using pGEM-T easy vectors and introduced into competent cells, JM109 (TOYOBO). A QIAprep Spin Miniprep Kit (QIAGEN) protocol was used to purify plasmid DNAs. Unidirectional sequencing of these cDNA clones was conducted on an ABI PRISM 3700 DNA Analyzer (Applied Biosystems).

### (5) Northern blotting analysis and RT-PCR

The SLURP-2 gene expression was evaluated by Northern hybridization and RT-PCR. For Northern hybridization, commercially available Human Digestive System 12-Lane MTN Blot (Clontech) was used. A 200-bp RNA derived from exon 1 to exon 3 was labeled with digoxigenin-11-dUTP using DIG RNA Labeling Kit (Roche), and used as a hybridization probe. The hybridized blot was washed twice with 2x SSC/0.1% SDS at room temperature and twice with 0.5x SSC/0.1% SDS at 68°C successively, and then detected according to the DIG system protocol.

For RT-PCR, poly A (+) RNAs from brain, heart, lungs, esophagus, stomach, small intestine, large intestine, rectum, liver, pancreas, spleen, kidneys, uterine, uterine cervix, testis, placenta, thymus, bone marrow, skeletal muscle, adult skin, fetal skin, cultured keratinocytes, and fibroblasts were used. Everything was purchased, except for the keratinocyte poly A (+) RNA, which was extracted from cultured keratinocytes. cDNA was synthesized using the ThermoScript™ RT-PCR System (Gibco BRL), and then amplified using primers designed for exon 1 and exon 3, 5'- GATTGAGGCAAGACTCCACG-3' (SEQ ID NO: 9) and 5'-CTGGCTGCAGCCGAAG-3' (SEQ ID NO: 10).

### (6) Southern blotting analysis of genomic DNA

Genomic DNA was isolated from human lymphocytes using a molecular cloning protocol, and digested once or twice with the following restriction enzymes: KpnI, EcoRI + BglII, and KpnI + BamHI (NEB) . The genomic DNA thus digested was separated on a 0.6% agarose gel and transferred to a positively charged Nylon membrane (ROCHE). By using DIG Easy Hybri (ROCHE) , this membrane was hybridized at 42°C for 16 hours with a DNA probe consisting of exon 3, which was labeled with DIG-11-dUTP using PCR DIG Labeling Mix (ROCHE). After hybridization, the membrane was washed once with 2x SSC/10% SDS at room temperature for five minutes, and twice with 0.5x SSC /10% SDS and 0.1x SSC /10% SDS at 68°C for 15 minutes each. A DIG Nucleic Acid Detection Kit (ROCHE) was used for detection.

### (7) Quantitative RT-PCR

Total RNA was extracted from skin biopsy specimens collected from the lesional and non-lesional areas of five patients with psoriasis vulgaris, and from a normal area of four healthy subjects. The total RNA was diluted to a final concentration of 2. 5 ng/µl. Five nanograms of total RNA was transcribed into cDNA. The cDNA was then amplified in 23 µl of reagent mixture (TaqMan One-Step RT-PCR Master Mix Reagents Kit, Applied Biosystems, Foster City, CA), comprising a uracil N-glycosylase-free 2x Master Mix, 40x MultiScribe, and RNase Inhibitor Mix, 300 nM each of a gene-specific forward primer 5'-GAGGGACTCCACCCACTGTGT-3' (SEQ ID NO: 11) and a reverse primer 5'-GCAGCCTATGTGGCACATCTT-3' (SEQ ID NO: 12), and 100 nM of a gene-specific FAM-labeled TaqMan probe 5'-CGGGTCCTCAGCAACACCGAGGAT-3' (SEQ ID NO: 13). As an internal positive control, 50 nM of 18S RNA-specific forward and reverse primers, and 50 nM of a VIC-labeled 18S RNA-specific Taqman probe were added. The resulting reactant' was subjected to reverse transcription at 48 °C for 30 minutes, incubated for ten minutes at 95 °C to activate AmpliTaq Gold, and then subjected to 50 cycles of a two-step amplification of one minute annealing/elongation at 62°C and 15 second denaturing at 95 °C. The total cDNA amount was standardized against the total amount of 18S RNA.

### [Example 1] Cloning of the SLURP-2 gene

Recent availability of gene microarray technology enables comparison and analysis of the comprehensive gene expression profiles in affected skin and in normal skin (Lockhart, D.J. et al. (1996) Nat. Biotechnol. 14: 1675-1680). The volume of data obtainable using this approach overcomes the limitations of analysis that investigates changes in single genes at the same time. Since diseased tissues contain information associated with the disease process, the present inventors anticipated that it would be valuable to construct gene expression profiles of skin tissues. Accordingly, the present inventors analyzed the expression profiles of large quantities of expressed sequence tags (ESTs) and known genes, using GeneChip arrays U95A, U95B, U95C, U95D, and U95E.

Specifically, the transcriptional changes in affected and non-affected skin areas of psoriasis patients were compared with normal controls by comprehensive analysis using the oligonucleotide arrays from Affymetrix Inc., which contain approximately 12,000 known genes and 48,000 unknown ESTs. The results showed 86 ESTs whose transcription was upregulated or downregulated by more than ten-folds in psoriasis lesions compared to normal tissues. One of these unknown ESTs (GenBank accession No. AI829641) was cloned.

### [Example 2] Isolation of the full-length cDNA and structural analysis of the SLURP-2 gene

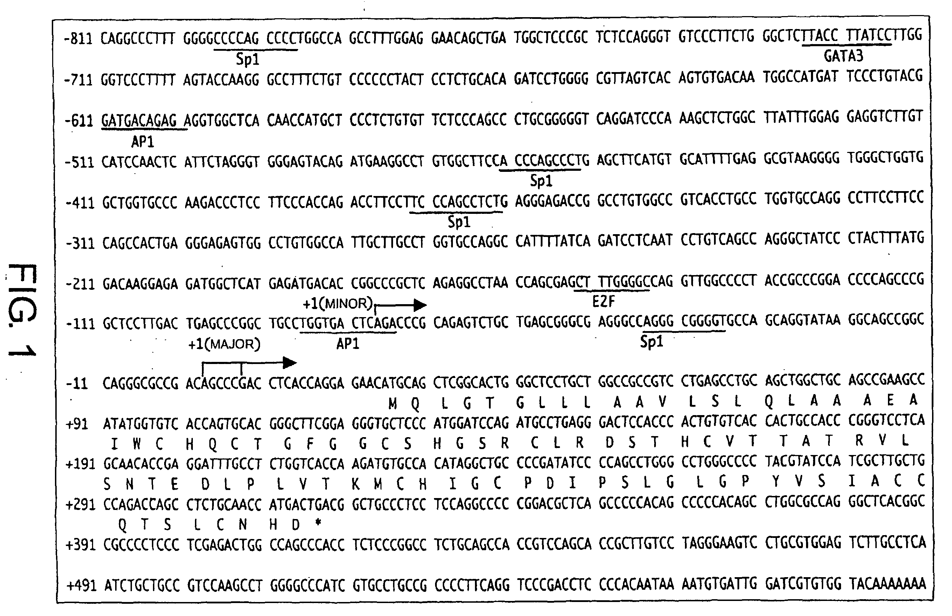

The present inventors isolated a full-length cDNA using RACE and oligo-capping (Fig. 1). Sequence analysis of the cDNA revealed that the gene is about 5.6 kb in length, and comprises three exons, and that its exon-intron boundaries comply with the GT-AG rule (Breathnach, R., and Chambon P. Organization an expression of eucaryotic split genes coding for proteins. (1981) Annu. Rev. Biochem. 50: 349-383) (Table 1).

**Table 1**

| Exon | | 5' Splice donor | | Intron | | 3' Splice acceptor | |
|---|---|---|---|---|---|---|---|
| No. | Size (bp) | Exon sequence | Intron sequence | No. | Size (bp) | Intron sequence | Exon sequence |
| 1 | 69 | CTGCAGCTGG | gtgagtccag | 1 | 4614 | atgtccgcag | CTGCAGCCGA |
| 2 | 294 | ACTGCCACCC | gtaagtggga | 2 | 294 | gcccactcag | GGGTCCTCAG |
| 3 | 403 | | | | | | |

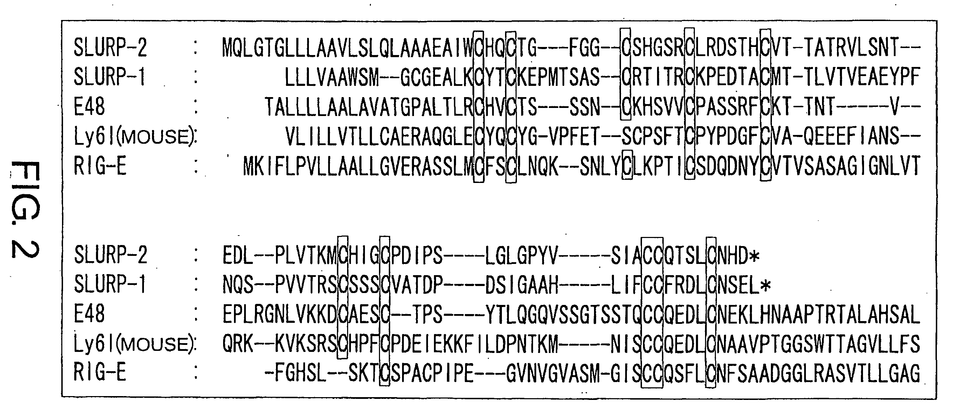

The open reading frame (ORF) encodes a protein of 97 amino acids comprising a Ly/uPAR domain that contains ten cysteine residues displayed in a conserved characteristic alignment pattern (Fig. 2). BLAST homology search results revealed a 29-31% identity to other members of the human and mouse Ly-6 superfamilies at the amino acid level. Furthermore, prediction of signal peptides using a SignalP predictor revealed that this protein comprises a signal peptide of 22 amino acids. GPI anchors were not found. These data indicate that the protein is a secretory protein.

### [Example 3] Identification of transcriptional initiation sites

Three transcriptional initiation sites were identified using RT-PCR and oligo-capping methods. To identify 5' ends, RT-PCR was conducted using gene-specific primers (forward primers were designed for an internal or a peripheral region of the initiator), and the transcriptional initiation site was shown to be at nucleotide position -81 (Fig. 1). The present inventors also used oligo-capping to determine the 5' ends, and the results showed that this gene comprises two transcriptional initiation sites. While eleven of the 20 clones were found to have a nucleotide at position +1, as shown in Fig. 1, nine clones had a nucleotide at position +5, which corresponds to the SMART RACE analysis 5' end. No clones were found to comprise a nucleotide at position -80.

### [Example 4] Analysis of the SLURP-2 gene promoter regions

The promoter regions were computer analyzed using TFSEARCH software. The results showed interesting promoter sites, as characterized in Fig. 1. While there was no TATA box or CAAT consensus sequence, three SP-1 binding sites were found in a GC-rich region. One SP-1 binding site was found at positions 37 to 46 downstream of the major transcriptional initiation site. Two E2F binding sites and one AP-1 binding site were also found. E2F and AP-1 are both known to be markers of cell proliferation. One GATA-3, which acts on T-cell differentiation, was also found.

### [Example 5] SLURP-2 gene expression analysis

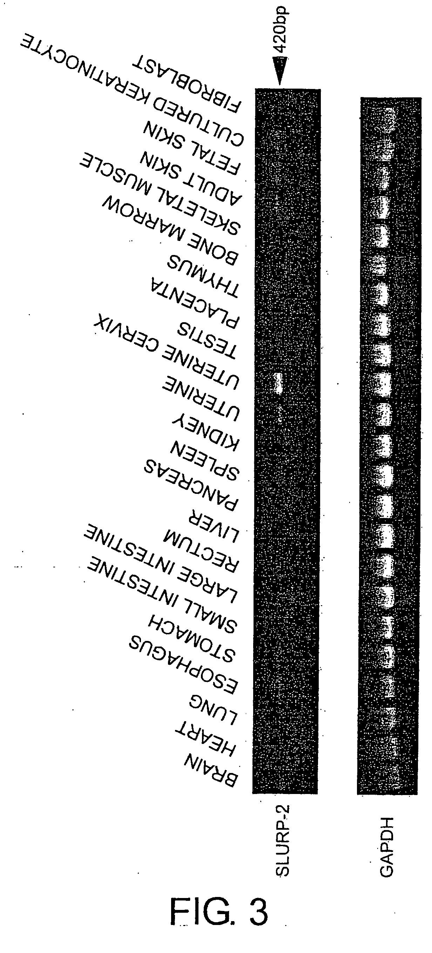

Expression of the SLURP-2 gene in various tissues was analyzed. The results of RT-PCR showed that the SLURP-2 gene is highly expressed in uterine cervix and esophagus, followed by adult and fetal skin, and keratinocytes. Low expression of the SLURP-2 gene was observed in brain, lung, stomach, small intestine, large intestine, rectum, uterus, and thymus (Fig. 3). SLURP-2 expression was not observed in spleen and bone marrow.

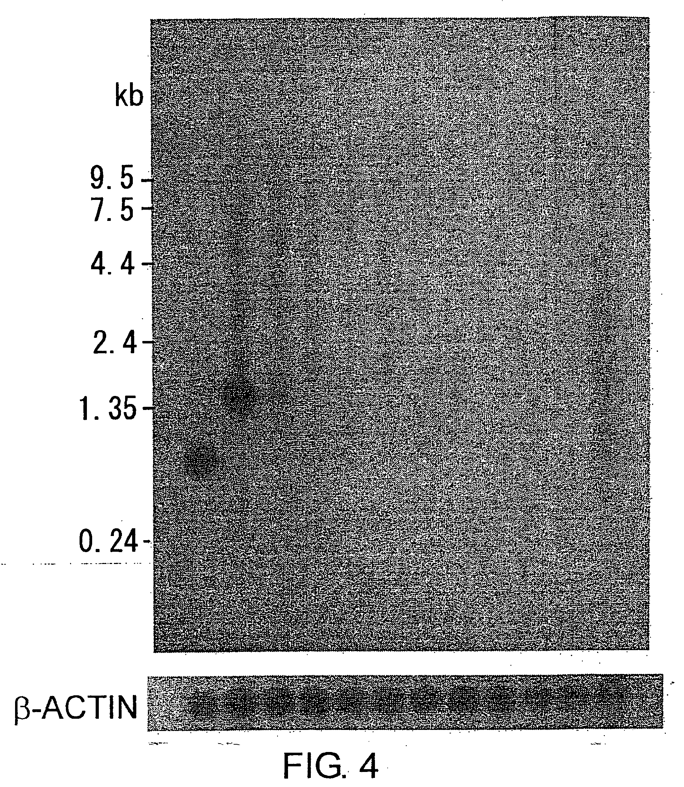

Northern hybridization using the Human digestive system 12 Lane (Clontech) exhibited SLURP-2 expression in esophagus, stomach, and duodenum (Fig. 4). Positive bands were detected at different positions; about 660 bp in esophagus and about 1, 600 bp in both stomach and duodenum. Moreover, the gene expression level also differs depending on the tissue, and the highest level was in the esophagus. Bands were not detected in the expression analyses of keratinocytes and human skin using Northern hybridization.

### [Example 6] Southern hybridization of genome

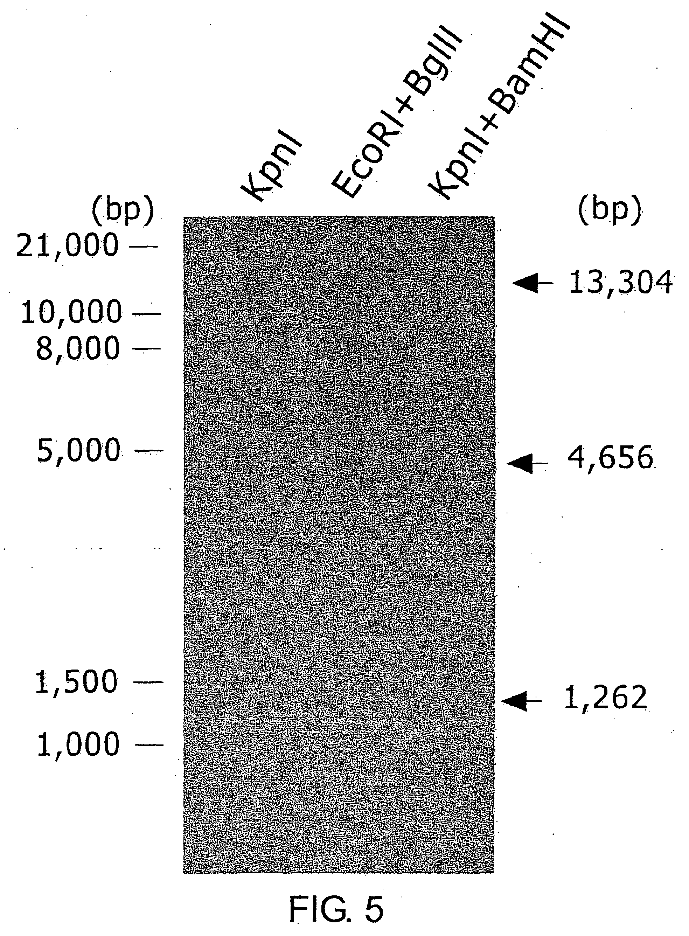

Southern hybridization analysis of human genomic DNA treated by single or double restriction digestion was carried out using a DIG-labeled exon 3 of the SLURP-2 gene as a probe. As a result, only a single band was detected at the position corresponding to the predicted size (Fig. 5). This result suggests that the SLURP-2 gene exists as a single gene in the human genome. Based on the genome sequence within ALO11976, the band sizes on the blot have all been accurately predicted.

### [Example 7] Quantitative RT-PCR

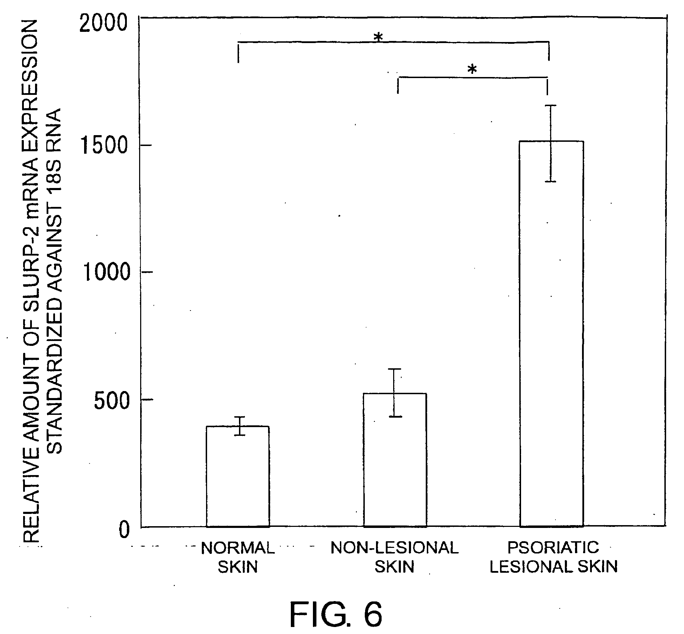

A relative and quantitative real-time RT-PCR analysis of the individual cDNAs derived from lesional skin (n= 5) and non-lesional skin of patients with psoriasis (n= 5) , and from normal skin of healthy subjects (n= 4) revealed that the SLURP-2 gene is upregulated in the lesional skin of psoriasis patients compared with their non-lesional skin, or that of the normal controls (p<0.0001, Fig. 6). Expression of the SLURP-2 gene in the lesional skin of psoriasis was 3.8-fold and 2.8-fold higher than those in the normal skin and in the non-lesional skin of psoriasis patients, respectively. The level of SLURP-2 gene expression in the non-lesional skin of psoriasis patients was almost the same as in normal skin.

The present inventors isolated the full-length cDNA of a novel human gene from cultured keratinocytes that encode a protein named SLURP-2, whose expression is upregulated in psoriatic lesions. This novel gene consists of three exons and two introns (580 bp in total) and comprises an ORF encoding 97 amino acids. The encoded amino acid sequence is distinct, and comprises ten cysteine residues displaying the same characteristic alignment pattern as the Ly-6/uPAR motif (Fig. 2) . However, this sequence does not comprise a GPI-anchor, which is another characteristic shared among most Ly-6 superfamily members. Recently, the Ly-6/uPAR superfamily was shown to have two subfamilies. Members of the first subfamily, such as CD59, E48 (Shan, X., Bourdear, A., Rhoton, A., Wells, D. E., et al. (1998) Characterization and mapping to human chromosome 8q24.3 of Ly-6-related gene 9804 encoding an apparent homologue of mouse TSA-1. J. Immunol. 160: 197-208), and RIG-E (Adermann K, Wattler F, Wattler S, Heine G, et al. (1999) Structural and phylogenetic characterization of human SLURP-1, the first secreted mammalian member of the Ly6/uPAR protein superfamily. Protein Sci. 8: 810-819), comprise ten cysteine residues as well as a GPI-anchor. Members of the second subfamily, such as SLURP-1 (secretory Ly-6/uPAR-rerated protein 1) and secretory snake venom and frog toxins, comprise eight to ten cysteine residues but no GPI-anchor. SLURP-1 is the first secretory protein found to belong to the mammalian Ly/uPAR superfamily (Shan, X., Bourdear; A., Rhoton, A., Wells, D. E., et al. (1998) Characterization and mapping to human chromosome 8q24.3 of Ly-6-related gene 9804 encoding an apparent homologue of mouse TSA-1. J. Immunol. 160: 197-208). A BLASTp homology search at the amino acid level also revealed that SLURP-2 is 34-28% identical to most of the Ly-6 superfamily members. These findings indicate that SLURP-2 is a new Ly-6/uPAR protein member and belongs to the second subfamily.

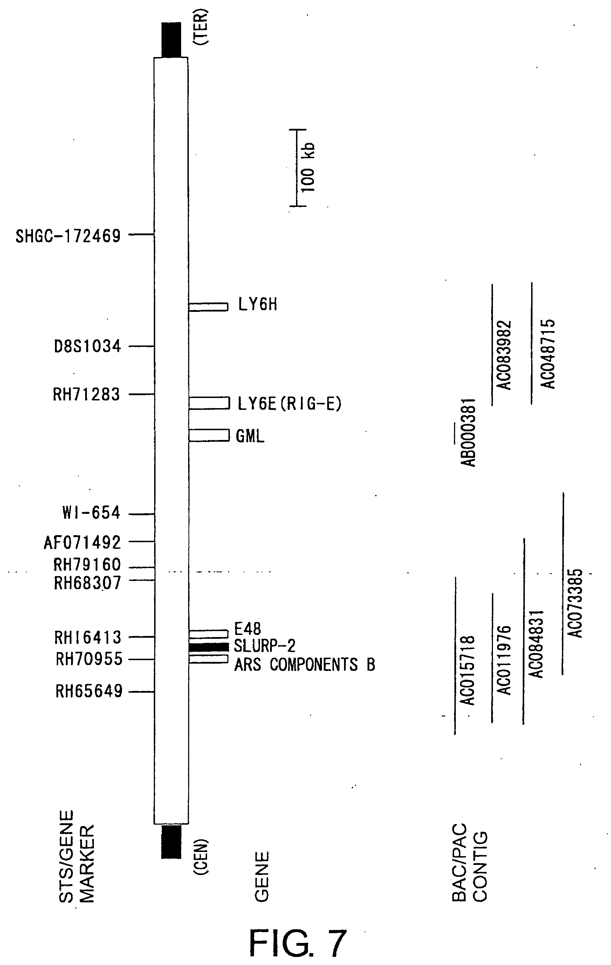

A BLASTn database search revealed that the AI829641 EST selected by the present inventors falls within the AI011976 clone sequence. This clone is mapped to 8q24. 3 corresponding to mouse chromosome 15, in which the Ly-6 superfamily members are clustered. Many of the human Ly-6 superfamily members are also clustered at this 8q24.3 region. Interestingly, according to the mapping data from Golden Path, 2001 DEC, ARS components B and E48, which are other members of the Ly-6 superfamily and are expressed in skin/keratinocytes, were mapped to approximately 50 kb within the SLURP-2 gene, and formed a small cluster, (Fig.7). E48 is found in SCC cell lines of head and neck, and is suggested to be identical to the cell adhesion molecule desmoglein III/dg4 (Brakenhoff, R. H., Gerretsen, M., Knipples, E. M. C., van Dijk, M., et al., (1995) The human E48 antigen, highly homologous with the murine Ly-6 antigen ThB, is a GPI-anchored molecule apparently involved in keratinocyte cell-cell adhesion. J. Cell Biol. 129: 1677-1689). Since the E48 mouse homolog is highly homologous to the EGF repeat of Notch family (Apostolopoulos, J., McKenzie, I. F. C., and Sandrin, M. S. (2000) LY6d-L, a cell surface ligand for mouse Ly6d. Immunity 12: 223-232), E48 is suggested to be involved in cell proliferation. Secretory Ly/uPAR related protein 1 (SLURP-1) is known to cause Mal de Meleda disease, which is an autosomal recessive palmoplantar keratoderma (Fischer, J., Bouadjar, B., Heilig, R., Huber, M., et al. (2001) Mutations I the gene encoding SLURP-1 in Mal de Meleda. Hum. Mol. Gen. 10: 875-880). The two Ly-6 members expressed in the skin are very likely to be involved in the hyperproliferation of keratinocytes.

The present inventors conducted an expression analysis using RT-PCR to show that the SLURP-2 gene is expressed mainly in epithelial tissues, such as skin. However, Northern blotting analysis of keratinocytes or human skin did not detect any bands. The very low expression of SLURP-2 gene in these tissues may explain why Northern blotting analysis did not detect any bands. The size and quantity of complete mRNAs differs between tissues, as shown in the Northern blotting analyses of esophagus and duodenum. The size was larger than the keratinocyte-derived mRNA, whose size was predicted using the main transcriptional initiation site determined using oligo-capping (Suzuki, Y., Yoshimoto-Nakagawa, K., Maruyama, K., Suyama, A., and Sugano, S. (1997) Construction and characterization of a full length-enriched and a 5'-end-enriched cDNA library. Gene 200: 149-156). However, the size of the mRNA in esophagus corresponded to that predicted using the minor transcriptional initiation site determined by RT-PCR. These findings suggest the possibility that the SLURP-2 gene is transcribed by alternative splicing at different transcriptional initiation sites.

RT-PCR expression analysis also revealed that the SLURP-2 gene is expressed in thymus, but not in bone marrow or spleen, which is another characteristic of SLURP-2 gene expression (Fig. 3) . Some Ly-6 superfamily members are known to be cell surface markers for the differentiation of hematopoietic cells and thymocytes. For example, Sca-1 (Ly6A/E) is used as a marker for hematopoietic stem cells (Spangrude G. J., Heimfeld S., and Weissman, I. L. (1988) Purification and characterization of mouse hematopoietic stem cells. Science. 241: 58-62; Spangrude G. and Jscollay, R. (1990) A simplified method for enrichment of mouse hematopoietic stem cells. Exp Hematol. 18: 920-926). Sca-2 and Ly-6G are markers for immature thymocytes and myeloid cells, respectively (Godfrey D. I., Masciantonio, M., Tucek C. L., Malin, M. A., Boyd, R. L., and Hugo, P. (1992) Thymic shared antigen-1: a novel thymocyte marker discriminating immature from mature thymocyte subsets. J Immunol. 148: 2006-2011; Fleming T. J., Fleming M. L., and Malek, T. R. (1993) Selective expression of Ly-6G on myeloid lineage cells in mouse bone marrow: RB6-8C5 mAb to granulocyte-differentiation antigen (Gr-1) detects members of the Ly-6 family. J Immunol. 151: 2399-2408), and Ly6C is a peripheral T cell activation antigen (McCormack, J. M., Leenen, P. J. M. and Walker, W. S. (1993) Macrophage progenitors from mouse bone marrow and spleen differ in their expression of the Ly-6C differentiation antigen. J. Immunol. 151: 6389-6398). However, such functions have not been found in human Ly-6 superfamily members. Moreover, the functions of the human Ly-6 superfamily members remain unclear, with the exception of CD59 and uPAR. The thymus is an organ for the maturation of T lymphocytes through their interaction with epithelial cells. The finding that the SLURP-2 gene is expressed in thymus and not in other blood-related tissues suggests that SLURP-2 is possibly related to epithelial cell components, or is a specific T cell antigen in thymus, or both.

Homology analysis using Swiss Prot revealed that SLURP-2 is 32% identical to mouse 4-1BB ligand (4-1BBL) at the amino acid level. 4-1BB is known to be an inducible T cell antigen, and belongs to the tumor necrosis factor (TNF) family, which is expressed in activated CD4 and CD8 (Smith, C. A., Davis, T., Anderson, D., Solam, L., et al. A receptor for tumor necrosis factor defines'an unusual family of cellular and viral proteins. Science 248: 1019-1023; Loetshcer, H., Pan, Y-C. E., Lahm, H-W., Gentz, R. et al. (1990) Molecular cloning and expression of the human 55 kd tumor necrosis factor receptor. Cell 61: 351-359; Schall, T. J., Lewis, M., Koller, K. J., Lee, A., et al. (1990) Molecular cloning and expression of a receptor for human tumor necrosis factor. Cell 61: 361-370). 4-1BB/4-1BBL is also known to induce the activation and differentiation of CD4+ and CD8+ cells (Vinay, D. S., and Kwon, B. S. (1998) Role of 4-1BB in immune responses. Sem. Immunol. 10: 481-489). TNF exhibits various functions including thymocyte proliferation and differentiation, T cell proliferation, IL-2R induction, and INF-γ production. Since SLURP-2 is a secretory protein, there is a possibility that it can act as a ligand for an unknown receptor functioning in T cell activation.

To isolate a gene associated with psoriasis vulgaris, the present inventors cloned the SLURP-2 gene based on microarray technology expression profiling results. Quantitative real-time RT-PCR analysis revealed that the SLURP-2 gene is significantly upregulated in psoriatic lesions compared to non-lesional skin and normal skin (Fig. 6). Promoter analysis indicated that some of the AP-1 and E2F sites are possibly related to the hyperproliferation of keratinocytes in psoriasis. Promoter analysis also identified an additional promoter site, GATA-3, far upstream of the promoter region. Interestingly, GATA-3 is a promoter that induces T cell differentiation (Vinay, D. S. and Kwon, B. S. (1998) Role of 4-1BB in immune responses. Sem. Immunol. 10: 481-489).

### Industrial Applicability

The present inventors isolated the SLURP-2 gene as a gene expressed in the lesional areas of inflammatory skin diseases. Thus, the gene can be used as a diagnostic marker for inflammatory skin diseases.

## Claims

1. A DNA of any one of (a) to (e) :
(a) a DNA encoding a polypeptide comprising the amino acid sequence of SEQ ID NO: 2;
(b) a DNA comprising the coding region of the nucleotide sequence of SEQ ID NO: 1;
(c) a DNA encoding a polypeptide comprising the amino acid sequence of SEQ ID NO: 2, wherein one or more amino acids are substituted, deleted, inserted, and/or added, and wherein said polypeptide is functionally equivalent to the polypeptide comprising the amino acid sequence of SEQ ID NO: 2;
(d) a DNA that hybridizes under stringent conditions to a DNA comprising the nucleotide sequence of SEQ ID NO: 1, and that encodes a polypeptide functionally equivalent to a polypeptide comprising the amino acid sequence of SEQ ID NO: 2; and
(e) a DNA that hybridizes under stringent conditions to a DNA comprising the nucleotide sequence of SEQ ID NO: 1.

2. A DNA encoding a partial peptide of a polypeptide comprising the amino acid sequence of SEQ ID NO: 2.

3. A polypeptide encoded by the DNA of claim 1 or 2.

4. A vector into which the DNA of claim 1 or 2 is inserted.

5. A transformed cell comprising the DNA of claim 1 or 2, or the vector of claim 4.

6. A method for producing the polypeptide of claim 3, wherein said method comprises the steps of:
culturing the transformed cell of claim 5; and
recovering the expressed polypeptide from said transformed cell, or culture supernatant thereof.

7. An antibody binding to the polypeptide of claim 3.

8. The antibody of claim 7, which is a monoclonal antibody.

9. An oligonucleotide comprising at least 15 nucleotides, which is complementary to the DNA of claim 1 or a complementary strand thereof.

10. A method of screening for a compound that binds to the polypeptide of claim 3, wherein said method comprises the steps of:
(a) contacting a test sample with said polypeptide;
(b) detecting binding activity between said polypeptide and said test sample; and
(c) selecting a compound having binding activity to said polypeptide.

11. A method of testing for an inflammatory skin disease, wherein said method comprises the steps of:
(a) preparing an RNA sample from a test subject;
(b) measuring, in said RNA sample, the amount of RNA that encodes the polypeptide of claim 3; and
(c) comparing the amount of RNA thus determined with a control sample.

12. A method of testing for an inflammatory skin disease, which comprises the steps of:
(a) preparing a cDNA sample from a test subject;
(b) measuring, in said cDNA sample, the amount of a cDNA that encodes the polypeptide of claim 3; and
(c) comparing the amount of cDNA thus determined with a control sample.

13. A method of testing for an inflammatory skin disease, wherein said method comprises the steps of:
(a) preparing a polypeptide sample from a test subject;
(b) measuring the amount of the polypeptide of claim 3 in said polypeptide sample; and
(c) comparing the amount of polypeptide thus determined with a control sample.

14. The method of any one of claims 11 to 13, wherein the inflammatory skin disease is psoriasis.

15. A test agent for an inflammatory skin disease, comprising the oligonucleotide of claim 9.

16. A test agent for an inflammatory skin disease, comprising the antibody of claim 7 or 8.

17. The test agent of claim 15 or 16, wherein the inflammatory skin disease is psoriasis.


SEQUENCE LISTING

<110> INOKO Hidetoshi

<120> INFLAMMATORY SKIN DISEASE-ASSOCIATED SLURP-2 GENE, AND USES
THEREOF

<130> C1-A0207P

<140>
<141>

<150> JP 2002-160285
<151> 2002-05-31

<160> 17

<170> PatentIn Ver. 2.1

<210> 1
<211> 590
<212> DNA
<213> Homo sapiens

<220>
<223> cDNA

<220>

<221> CDS

<222> (23)..(316)

<400> 1

agcccgacct caccaggaga ac atg cag ctc ggc act ggg ctc ctg ctg gcc 52

Met Gln Leu Gly Thr Gly Leu Leu Leu Ala

1               5                   10

gcc gtc ctg agc ctg cag ctg gct gca gcc gaa gcc ata tgg tgt cac 100

Ala Val Leu Ser Leu Gln Leu Ala Ala Ala Glu Ala Ile Trp Cys His

                15                  20                  25

cag tgc acg ggc ttc gga ggg tgc tcc cat gga tcc aga tgc ctg agg 148

Gln Cys Thr Gly Phe Gly Gly Cys Ser His Gly Ser Arg Cys Leu Arg

            30                  35                  40

gac tcc acc cac tgt gtc acc act gcc acc cgg gtc ctc agc aac acc 196

Asp Ser Thr His Cys Val Thr Thr Ala Thr Arg Val Leu Ser Asn Thr

        45                  50                  55

gag gat ttg cct ctg gtc acc aag atg tgc cac ata ggc tgc ccc gat 244

Glu Asp Leu Pro Leu Val Thr Lys Met Cys His Ile Gly Cys Pro Asp

    60                  65                  70

atc ccc agc ctg ggc ctg ggc ccc tac gta tcc atc gct tgc tgc cag 292
Ile Pro Ser Leu Gly Leu Gly Pro Tyr Val Ser Ile Ala Cys Cys Gln
75                  80                  85                  90

acc agc ctc tgc aac cat gac tga cggctgccct cctccaggcc cccggacgct 346
Thr Ser Leu Cys Asn His Asp
                95

cagcccccac agcccccaca gcctggcgcc agggctcacg gccgcccctc cctcgagact 406

ggccagccca cctctcccgg cctctgcagc caccgtccag caccgcttgt cctagggaag 466

tcctgcgtgg agtcttgcct caatctgctg ccgtccaagc ctggggccca tcgtgcctgc 526

cgccccttca ggtcccgacc tccccacaat aaaatgtgat tggatcgtgt ggtacaaaaa 586

aaaa 590

<210> 2
<211> 97
<212> PRT
<213> Homo sapiens
<223> cDNA

<400> 2

Met Gln Leu Gly Thr Gly Leu Leu Leu Ala Ala Val Leu Ser Leu Gln
1               5                   10                  15
Leu Ala Ala Ala Glu Ala Ile Trp Cys His Gln Cys Thr Gly Phe Gly
            20                  25                  30
Gly Cys Ser His Gly Ser Arg Cys Leu Arg Asp Ser Thr His Cys Val
        35                  40                  45
Thr Thr Ala Thr Arg Val Leu Ser Asn Thr Glu Asp Leu Pro Leu Val
    50                  55                  60
Thr Lys Met Cys His Ile Gly Cys Pro Asp Ile Pro Ser Leu Gly Leu
65                  70                  75                  80
Gly Pro Tyr Val Ser Ile Ala Cys Cys Gln Thr Ser Leu Cys Asn His
                85                  90                  95
Asp

<210> 3
<211> 23
<212> DNA
<213> Artificial Sequence

<220>
<223> Description of Artificial Sequence:an artificially
      synthesized primer sequence

<400> 3
ctaggacaag cggtgctgga cgg                                          23

<210> 4
<211> 23
<212> DNA
<213> Artificial Sequence

<220>
<223> Description of Artificial Sequence:an artificially
      synthesized primer sequence

<400> 4
ctaggacaag cggtgctgga cgg                                              23

<210> 5
<211> 21
<212> DNA
<213> Artificial Sequence

<220>
<223> Description of Artificial Sequence:an artificially
      synthesized primer sequence

<400> 5
taggacaagc ggtgctggac g                                                21

<210> 6

<211> 23

<212> DNA

<213> Artificial Sequence

<220>

<223> Description of Artificial Sequence:an artificially

synthesized primer sequence

<400> 6

ggcagcaagc gatggatacg tag 23

<210> 7

<211> 23

<212> DNA

<213> Artificial Sequence

<220>

<223> Description of Artificial Sequence:an artificially

synthesized primer sequence

<400> 7

ggcagcaagc gatggatacg tag 23

<210> 8

<211> 20

<212> DNA

<213> Artificial Sequence

<220>

<223> Description of Artificial Sequence:an artificially

synthesized primer sequence

<400> 8

cagtgccgag ctgcatgttc 20

<210> 9

<211> 20

<212> DNA

<213> Artificial Sequence

<220>

<223> Description of Artificial Sequence:an artificially

synthesized primer sequence

<400> 9

gattgaggca agactccacg 20

<210> 10

<211> 16

<212> DNA

<213> Artificial Sequence

<220>

<223> Description of Artificial Sequence:an artificially

synthesized primer sequence

<400> 10

ctggctgcag ccgaag 16

<210> 11

<211> 21

<212> DNA

<213> Artificial Sequence

<220>

<223> Description of Artificial Sequence:an artificially

synthesized primer sequence

<400> 11

gagggactcc acccactgtg t 21

<210> 12

<211> 21

<212> DNA

<213> Artificial Sequence

<220>

<223> Description of Artificial Sequence:an artificially
      synthesized primer sequence

<400> 12
gcagcctatg tggcacatct t                                                21

<210> 13

<211> 24

<212> DNA

<213> Artificial Sequence

<220>

<223> Description of Artificial Sequence:Taq Man Probe sequence

<400> 13
cgggtcctca gcaacaccga ggat                                             24

<210> 14

<211> 95

<212> PRT

<213> Homo sapiens

<400> 14

Leu Leu Leu Val Ala Ala Trp Ser Met Gly Cys Gly Glu Ala Leu Lys
1               5                   10                  15

Cys Tyr Thr Cys Lys Glu Pro Met Thr Ser Ala Ser Cys Arg Thr Ile
            20                  25                  30

Thr Arg Cys Lys Pro Glu Asp Thr Ala Cys Met Thr Thr Leu Val Thr
        35                  40                  45

Val Glu Ala Glu Tyr Pro Phe Asn Gln Ser Pro Val Val Thr Arg Ser
    50                  55                  60

Cys Ser Ser Ser Cys Val Ala Thr Asp Pro Asp Ser Ile Gly Ala Ala
65                  70                  75                  80

His Leu Ile Phe Cys Cys Phe Arg Asp Leu Cys Asn Ser Glu Leu
                85                  90                  95

<210> 15

<211> 109

<212> PRT

<213> Homo sapiens

<400> 15

Thr Ala Leu Leu Leu Leu Ala Ala Leu Ala Val Ala Thr Gly Pro Ala
1               5                   10                  15

Leu Thr Leu Arg Cys His Val Cys Thr Ser Ser Ser Asn Cys Lys His
                20                  25                  30

Ser Val Val Cys Pro Ala Ser Ser Arg Phe Cys Lys Thr Thr Asn Thr
            35                  40                  45

Val Glu Pro Leu Arg Gly Asn Leu Val Lys Lys Asp Cys Ala Glu Ser
    50                  55                  60

Cys Thr Pro Ser Tyr Thr Leu Gln Gly Gln Val Ser Ser Gly Thr Ser
65                  70                  75                  80

Ser Thr Gln Cys Cys Gln Glu Asp Leu Cys Asn Glu Lys Leu His Asn
                85                  90                  95

Ala Ala Pro Thr Arg Thr Ala Leu Ala His Ser Ala Leu
            100                 105

<210> 16

<211> 113

<212> PRT

<213> Mus musculus

<400> 16

Val Leu Ile Leu Leu Val Thr Leu Leu Cys Ala Glu Arg Ala Gln Gly

1               5                   10                  15

Leu Glu Cys Tyr Gln Cys Tyr Gly Val Pro Phe Glu Thr Ser Cys Pro

            20                  25                  30

Ser Phe Thr Cys Pro Tyr Pro Asp Gly Phe Cys Val Ala Gln Glu Glu

        35                  40                  45

Glu Phe Ile Ala Asn Ser Gln Arg Lys Lys Val Lys Ser Arg Ser Cys

    50                  55                  60

His Pro Phe Cys Pro Asp Glu Ile Glu Lys Lys Phe Ile Leu Asp Pro

65                  70                  75                  80

Asn Thr Lys Met Asn Ile Ser Cys Cys Gln Glu Asp Leu Cys Asn Ala

                85                  90                  95

Ala Val Pro Thr Gly Gly Ser Trp Thr Thr Ala Gly Val Leu Leu Phe
            100                 105                 110
Ser

<210> 17
<211> 117
<212> PRT
<213> Homo sapiens

<400> 17
Met Lys Ile Phe Leu Pro Val Leu Leu Ala Ala Leu Leu Gly Val Glu
1               5                   10                  15
Arg Ala Ser Ser Leu Met Cys Phe Ser Cys Leu Asn Gln Lys Ser Asn
            20                  25                  30
Leu Tyr Cys Leu Lys Pro Thr Ile Cys Ser Asp Gln Asp Asn Tyr Cys
        35                  40                  45
Val Thr Val Ser Ala Ser Ala Gly Ile Gly Asn Leu Val Thr Phe Gly
    50                  55                  60
His Ser Leu Ser Lys Thr Cys Ser Pro Ala Cys Pro Ile Pro Glu Gly
65                  70                  75                  80

Val Asn Val Gly Val Ala Ser Met Gly Ile Ser Cys Cys Gln Ser Phe
                85                  90                  95
Leu Cys Asn Phe Ser Ala Ala Asp Gly Gly Leu Arg Ala Ser Val Thr
            100                 105                 110
Leu Leu Gly Ala Gly
        115